(19) Europäisches Patentamt / European Patent Office / Office européen des brevets

(11) **EP 1 843 799 B1**

(12) **EUROPEAN PATENT SPECIFICATION**

(45) Date of publication and mention
of the grant of the patent:
**25.02.2015 Bulletin 2015/09**

(21) Application number: **06733786.5**

(22) Date of filing: **23.01.2006**

(51) Int Cl.:
***A61L 15/60*** *(2006.01)*

(86) International application number:
**PCT/US2006/002112**

(87) International publication number:
**WO 2006/083583 (10.08.2006 Gazette 2006/32)**

(54) **ABSORBENT STRUCTURE WITH IMPROVED WATER-SWELLABLE MATERIAL**

SAUGFÄHIGE STRUKTUR MIT VERBESSERTEM WASSERQUELLBAREM MATERIAL

STRUCTURE ABSORBANTE COMPRENANT UNE MATIERE AMELIOREE ABSORBANT L'EAU

(84) Designated Contracting States:
**AT BE BG CH CY CZ DE DK EE ES FI FR GB GR HU IE IS IT LI LT LU LV MC NL PL PT RO SE SI SK TR**

(30) Priority: **04.02.2005 US 650291 P**

(43) Date of publication of application:
**17.10.2007 Bulletin 2007/42**

(73) Proprietor: **THE PROCTER & GAMBLE COMPANY**
**Cincinnati, OH 45202 (US)**

(72) Inventors:
  • **SCHMIDT, Mattias**
    **65510 Idstein (DE)**
  • **MEYER, Axel**
    **60486 Frankfurt (DE)**
  • **FOSSUM, Renae, Dianna**
    **Middletown, Ohio 45044 (US)**
  • **GOLDMAN, Stephen, Allen**
    **Cincinnati, Ohio 45242 (US)**
  • **EHRNSPERGER, Bruno, Johannes**
    **Mason, Ohio 45040 (US)**
  • **RIEGEL, Ulrich**
    **66849 Landstuhl (DE)**
  • **DANIEL, Thomas, Dr.**
    **67165 Waldsee (DE)**
  • **BRUHNS, Stefan, Dr.**
    **86165 Mannheim (DE)**
  • **ELLIOT, Mark, BSc**
    **67063 Ludwigshafen (DE)**

(74) Representative: **Heide, Ute et al**
**Procter & Gamble Service GmbH**
**IP Department**
**Frankfurter Strasse 145**
**61476 Kronberg im Taunus (DE)**

(56) References cited:
EP-A- 1 433 450          WO-A-00/62922
WO-A-03/043670          WO-A-2005/014064
WO-A-2006/042704          WO-A1-99/57201
US-A- 4 392 908          US-A- 5 061 424
US-A- 5 634 572          US-A- 5 731 365
US-A- 5 851 672          US-A- 5 883 158
US-A1- 2002 128 618

**Description**

FIELD OF THE INVENTION

This invention relates to improved absorbent structures containing improved water-swellable material that can significantly withstand deformation by an external pressure, thus showing improved liquid handling properties. In particular, this invention relates to absorbent structures comprising water-swellable material with an improved absorbent capacity/permeability balance.

This invention also relates to absorbent structures comprising water-swellable material, that comprises water-swellable polymers and elastomeric polymers, said material being typically in the form of particles, which comprise a core of water-swellable polymer(s) and a shell of said elastomeric polymer(s), whereby the water-swellable material is such that it can withstand deformation due to external pressure.

The invention also relates to diapers, adult incontinence articles and sanitary napkins comprising said absorbent structure of the invention.

BACKGROUND OF THE INVENTION

An important component of disposable absorbent articles such as diapers is an absorbent core structure comprising water-swellable polymers, typically hydrogel-forming water-swellable polymers, also referred to as absorbent gelling material, AGM, or super-absorbent polymers, or SAP's. This polymer material ensures that large amounts of bodily fluids, e.g., urine, can be absorbed by the article during its use and locked away, thus providing low rewet and good skin dryness.

Especially useful water-swellable polymers or SAP's are often made by initially polymerizing unsaturated carboxylic acids or derivatives thereof, such as acrylic acid, alkali metal (e.g., sodium and/or potassium) or ammonium salts of acrylic acid, alkyl acrylates, and the like in the presence of relatively small amounts of di- or poly-functional monomers such as N,N'-methylenebisacrylamide, trimethylolpropane triacrylate, ethylene glycol di(meth)acrylate, or triallylamine. The di- or poly-functional monomer materials serve to lightly cross-link the polymer chains thereby rendering them water-insoluble, yet water-swellable. These lightly crosslinked absorbent polymers contain a multiplicity of carboxylate groups attached to the polymer backbone. It is generally believed, that the neutralized carboxylate groups generate an osmotic driving force for the absorption of body fluids by the crosslinked polymer network.

In addition, the polymer particles are often treated as to form a surface crosslinked layer on the outer surface in order to improve their properties in particular for application in baby diapers.

Water-swellable (hydrogel-forming) polymers useful as absorbents in absorbent members and articles such as disposable diapers need to have adequately high sorption capacity, as well as adequately high gel strength. Sorption capacity needs to be sufficiently high to enable the absorbent polymer to absorb significant amounts of the aqueous body fluids encountered during use of the absorbent article. Together with other properties of the gel, gel strength relates to the tendency of the swollen polymer particles to resist deformation under an applied stress. The gel strength needs to be high enough in the absorbent member or article, to reduce deformation and to avoid that the capillary void spaces between the particles are filled to an unacceptable degree, causing so-called gel blocking. This gel-blocking inhibits the rate of fluid uptake or the fluid distribution, i.e., once gel-blocking occurs, it can substantially impede the distribution of fluids to relatively dry zones or regions in the absorbent article and leakage from the absorbent article can take place well before the water-swellable polymer particles are fully saturated or before the fluid can diffuse or wick past the "blocking" particles into the rest of the absorbent article. Thus, it is important that the water-swellable polymers (when incorporated in an absorbent structure or article) have a high resistance against deformation thus maintaining a high wet-porosity, thus yielding high permeability for fluid transport through the swollen gel bed.

It is known in the art that absorbent polymers with relatively high permeability can be made by increasing the level of internal crosslinking and/or surface crosslinking, which increases the resistance of the swollen gel against deformation by an external pressure such as the pressure caused by the wearer, but this typically also reduces the absorbent capacity of the gel undesirably. To date, the manufacturer of water-swellable polymers will thus always have to select the surface crosslinking levels and internal cross-linking levels depending on the desired absorbent capacity and permeability.

It is a significant draw-back of this conventional approach that the absorbent capacity has to be sacrificed in order to gain permeability. The lower absorbent capacity must be compensated by higher dosage of the absorbent polymer in hygiene articles which for example leads to difficulties with the core integrity of a diaper or sanitary napkin during wear. Hence, special, technically challenging and expensive fixation technologies are required to overcome this issue and in addition higher costs are incurred by the required higher dosing level of the absorbent polymer itself.

The surface crosslinked water-swellable polymer particles are often constrained by their surface-crosslinked surface layer and cannot absorb or swell sufficiently; and also, the surface-crosslinked surface layer is not strong enough to withstand the stresses of swelling or the stresses associated with performance under load.

As a result thereof the surface-crosslinked surface layers of such water-swellable polymers, as used in the art, typically break when the polymer swells significantly. Often these surface-crosslinked water-swellable polymers deform significantly in use thus leading to relatively low porosity and permeability of the gel bed in the wet state.

Without wishing to be bound by any theory it is believed that the tangential forces that determine the stability against deformation are limited by the breaking of the shells or coatings.

The inventors have now found that the change in the absorbent capacity of the water-swellable material when it is submitted to a grinding method, is a measure to determine whether the original water-swellable material is such that it exerts a pressure, which is high enough to ensure a much improved permeability of the water-swellable material (when swollen), providing ultimately an improved absorbent capacity/permeability balance in use and an ultimately improved performance in use.

The inventors have also found a way to provide improved absorbent structures with improved water-swellable material which improved resistance against deformation when swollen and which provides an improved stability against external pressure, even when swollen. The material typically comprises particles of water-swellable polymers with a specific shell, which creates an internal pressure, which is exerted onto the water-swellable polymers within this shell. Without wishing to be bound by any theory, it is believed that if this internal pressure is significantly higher than the external pressure, e.g., the pressure exerted by the wearer of an absorbent article that comprises water swellable material, the shell will provide the stability of the particles against deformation, as it will try to minimize the energy by assuming a round shape as much as possible. It is believed that the internal pressure in the water-swellable material should be at least 50% higher than the typical external pressure exerted onto the water-swellable material, based on the average external pressure in use in absorbent articles. The inventors found thus that the internal pressure created by the shell should therefore preferably be in the range of about 0.45 psi (21.55 Pa) to about 1.05 psi (50.27 Pa), especially for water swellable materials that are used in absorbent articles such as baby diapers.

Just as the known surface-crosslinked water-swellable polymers described and available in the industry, comprising a surface-crosslinked outer surface, the shell of the water-swellable polymer particles of the water-swellable material herein will typically reduce the absorbent capacity of the water-swellable material to some degree, however, an improved balance is obtained with the water-swellable materials herein, due to the high pressure resistance of the shell whilst having a high expandability, allowing high absorbent capacity. Thus, the absorbent structures of the invention, comprising the improved water-swellable material herein, have an improved balance between absorbent capacity and permeability.

SUMMARY OF THE INVENTION

In a first embodiment, the invention provides an absorbent structure for use in an absorbent article, said absorbent structure comprising a water-swellable, said water-swellable material having an absorbent capacity of at least about 20g/g (as measured in the 4-hour CCRC test) and a QUICS value calculated therefrom, as defined herein, whereby said QUICS is at least 15, wherein said water-swellable material comprises water-swellable particles with a core, containing water- swellable polymer(s) and a shell, containing elastomeric polymer(s) and wherein said elastomeric polymer(s) are polyetherpolyurethanes comprising alkylene oxide units on at least one of a group selected from polymer main chains and polymer side chains.

The absorbent structure may be an absorbent article, or part of or incorporated in an absorbent article, such as a diaper, adult incontinence product, sanitary napkin. For example, it may be the storage layer of such an article, and it may have a density of at least 0.4 g/cm$^3$, and/or it may comprise less than 20% by weight (of the water-swellable material) of absorbent fibrous material.

BRIEF DESCRIPTION OF THE DRAWINGS

Fig. 1 is a schematic view of the permeability equipment setup.
Fig. 2 is a detailed view of the SFC cylinder/plunger apparatus.
Fig. 3 is a view of the SFC plunger details.

DETAILED DESCRIPTION Absorbent structures

"Absorbent structure" refers to any three dimensional structure, comprising at least water-swellable material, useful to absorb and retain liquids, such as urine, menses, or blood.

"Absorbent article" refers to devices that absorb and retain liquids (such as blood, menses and urine), and more specifically, refers to devices that are placed against or in proximity to the body of the wearer to absorb and contain the various exudates discharged from the body. Absorbent articles include but are not limited to diapers, including training pants, adult incontinence briefs, diaper holders and liners, sanitary napkins and the like.

"Diaper" refers to an absorbent article generally worn by infants and incontinent persons about the lower torso.

"Disposable" is used herein to describe articles that are generally not intended to be laundered or otherwise restored or reused (i.e., they are intended to be discarded after a single use and, preferably, to be recycled, composted or otherwise disposed of in an environmentally compatible manner).

The absorbent structure typically comprises the water-swellable material herein and a structuring material, such as a core wrap or wrapping material, support layer for the water-swellable material or structuring agent such as described below.

The absorbent structure is typically, or forms typically part of, an absorbent article, and preferably disposable absorbent articles, such as preferably sanitary napkins, panty liners, and more preferably adult incontinence products, diapers, and training pants.

If the absorbent structure is part of a disposable absorbent article, then the absorbent structure of the invention is typically that part of an absorbent article which serves to store and/or acquire bodily fluids, the absorbent structure may be the storage layer of an absorbent article, or the acquisition layer, or both, either as two or more layers or as unitary structure.

The absorbent structure may be a structure that consists of the water-swellable material and that is then shaped into the required three-dimensional structure, or preferably, it may comprise additional components, such as those used in the art for absorbent structures.

Preferred is that the absorbent structure also comprise one or more support or wrapping materials, such as foams, films, woven webs and/or nonwoven webs, as known in the art, such as spunbond, meltblown and/ or carded nonwovens. One preferred material is a so-called SMS material, comprising a spunbonded, a melt-blown and a further spunbonded layer. Highly preferred are permanently hydrophilic nonwovens, and in particular nonwovens with durably hydrophilic coatings. An alternative preferred material comprises a SMMS-structure. The top layer and the bottom layer may be provided from two or more separate sheets of materials or they may be alternatively provided from a unitary sheet of material

Preferred non-woven materials are provided from synthetic fibers, such as PE, PET and most preferably PP. As the polymers used for nonwoven production are inherently hydrophobic, they are preferably coated with hydrophilic coatings, e.g., coated with nanoparticles, as known in the art.

Preferred nonwoven materials and absorbent structures using such materials are described in, for example, co-pending applications US2004/03625, US2004/03624, and US2004/03623 and in US 2004/0162536, EP1403419-A, WO2002/0192366, EP 1470281-A and EP1470282-A.

The absorbent structure may also comprise a structuring agent or matrix agent, such as absorbent fibrous material, such as airfelt fibers, and/or adhesive, which each may serve to immobilize the water-swellable material.

Because the water-swellable material herein has an excellent permeability, even when swollen, there is no need for large amounts of structuring agents, such as absorbent fibrous material (airfelt), as normally used in the art.

Thus, preferably a relatively low amount or no absorbent fibrous (cellulose) material is used in the absorbent structure. Thus, it may be preferred that said structure herein comprises large amounts of the water-swellable material herein and only very little or no absorbent (cellulose) fibers, preferably less than 20% by weight of the water-swellable material, or even less than 10% by weight of the water-swellable material, or even less than 5% by weight.

Preferred absorbent structures herein comprise a layer of a substrate material such as the core-wrap materials described herein, and thereon a water-swellable material layer, optionally as a discontinuous layer, and thereon a layer of an adhesive or thermoplastic material or preferably a (fibrous) thermoplastic adhesive material, which is laid down onto the layer of water-swellable material. Preferred may be that the thermoplastic or adhesive layer is then in direct contact with the water-swellable material, but also partially in direct contact with the substrate layer, where the substrate layer is not covered by the absorbent polymeric material. This imparts an essentially three-dimensional structure to the (fibrous) layer of thermoplastic or adhesive material, which in itself is essentially a two-dimensional structure of relatively small thickness (in z-direction), as compared to the extension in x- and y-direction.

Thereby, the thermoplastic or adhesive material provides cavities to hold the water-swellable material and thereby immobilizes this material. In a further aspect, the thermoplastic or adhesive material bonds to the substrate and thus affixes the water-swellable material to the substrate.

In this embodiment, it may be preferred that no absorbent fibrous material is present in the absorbent structure.

The thermoplastic composition may comprise, in its entirety, a single thermoplastic polymer or a blend of thermoplastic polymers, having a softening point, as determined by the ASTM Method D-36-95 "Ring and Ball", in the range between 50°C and 300°C, or alternatively the thermoplastic composition may be a hot melt adhesive comprising at least one thermoplastic polymer in combination with other thermoplastic diluents such as tackifying resins, plasticizers and additives such as antioxidants.

The thermoplastic polymer has typically a molecular weight (Mw) of more than 10,000 and a glass transition temperature (Tg) usually below room temperature. A wide variety of thermoplastic polymers are suitable for use in the present invention. Such thermoplastic polymers are preferably water insensitive. Exemplary polymers are (styrenic) block copolymers including A-B-A triblock structures, A-B diblock structures and (A-B)n radial block copolymer structures wherein the A blocks are non-elastomeric polymer blocks, typically comprising polystyrene, and the B blocks are unsaturated conjugated diene or (partly) hydrogenated versions of such. The B block is typically isoprene, butadiene, ethylene/butylene (hydrogenated butadiene), ethylene/propylene (hydrogenated isoprene), and mixtures thereof.

Other suitable thermoplastic polymers that may be employed are metallocene polyolefins, which are ethylene polymers prepared using single-site or metallocene catalysts. Therein, at least one comonomer can be polymerized with ethylene to make a copolymer, terpolymer or higher order polymer. Also applicable are amorphous polyolefins or amorphous polyalphaolefins (APAO) which are homopolymers, copolymers or terpolymers of C2 to C8 alphaolefins.

The resin has typically a Mw below 5,000 and a Tg usually above room temperature, typical concentrations of the resin in a hot melt are in the range of 30 - 60%. The plasticizer has a low Mw of typically less than 1,000 and a Tg below room temperature, a typical concentration is 0 -15%.

Preferably the adhesive is present in the forms of fibres throughout the core, i.e., the adhesive is fiberized or fibrous.

Preferably, the fibres will preferably have an average thickness of 1 - 50 micrometer and an average length of 5 mm to 50 cm.

Preferably, the absorbent structure, in particular when no or little absorbent fibres are present, as described above, has a density greater than about 0.4 g/cm$^3$. Preferably, the density is greater than about 0.5 g/cm$^3$, more preferably greater than about 0.6 g/cm$^3$.

Preferred absorbent structures can, for example, be made as follows:

a) providing a substrate material that can serve as a wrapping material;

b) depositing the water-swellable material herein onto a first surface of the substrate material, preferably in a pattern comprising at least one zone which is substantially free of water-swellable material, and the pattern comprising at least one zone comprising water-swellable material, preferably such that openings are formed between the separate zones with water-swellable material;

c) depositing a thermoplastic material onto the first surface of the substrate material and the water-swellable material, such that portions of the thermoplastic material are in direct contact with the first surface of the substrate and portions of the thermoplastic material are in direct contact with the water-swellable material; and

d) then typically closing the above by folding the substrate material over, or by placing another substrate matter over the above.

The absorbent structure may comprise an acquisition layer and a storage layer, which may have the same dimensions, however it may be preferred that the acquisition layer is laterally centered on the storage layer with the same lateral width but a shorter longitudinal length than storage layer. The acquisition layer may also be narrower than the storage layer while remaining centered thereon. Said another way, the acquisition layer suitably has an area ratio with respect to storage layer of 1.0, but the area ratio may preferably be less than 1.0, e.g., less than about 0.75, or more preferably less than about 0.50.

For absorbent structures and absorbent articles designed for absorption of urine, it may be preferred that the acquisition layer is longitudinally shorter than the storage layer and positioned such that more than 50% of its longitudinal length is forward of transverse axis of the absorbent structure or of the absorbent article herein. This positioning is desirable so as to place acquisition layer under the point where urine is most likely to first contact absorbent structure or absorbent article.

Also, the absorbent core, or the acquisition layer and/or storage layer thereof, may comprise an uneven distribution of water-swellable material basis weight in one or both of the machine and cross directions. Such uneven basis weight distribution may be advantageously applied in order to provide extra, predetermined, localized absorbent capacity to the absorbent structure or absorbent article.

The absorbent structure of the invention may be, or may be part of an absorbent article, typically it may be the absorbent core of an absorbent article, or the storage layer and/or acquisition layer of such an article.

Preferred (disposable) absorbent article comprising the absorbent structure of the invention are sanitary napkins, panty liners, adult incontinence products and infant diapers or training or pull-on pants, whereby articles which serve to absorb urine, e.g., adult incontinence products, diapers and training or pull-on pants are the most preferred articles herein.

Preferred articles herein have a topsheet and a backsheet, which each have a front region, back region and crotch region, positioned therein between. The absorbent structure of the invention is typically positioned in between the topsheet and backsheet. Preferred backsheets are vapor pervious but liquid impervious. Preferred topsheet materials are at least partially hydrophilic; preferred are also so-called apertured topsheets. Preferred may be that the topsheet comprises a skin care composition, e.g., a lotion.

These preferred absorbent articles typically comprise a liquid impervious (but preferably air or water vapor pervious) backsheet, a fluid pervious topsheet joined to, or otherwise associated with the backsheet. Such articles are well known in the art and fully disclosed in various documents mentioned throughout the description.

Because the water-swellable material herein has a very high absorbency capacity, it is possible to use only low levels of this material in the absorbent articles herein. Preferred are thus thin absorbent articles, such as adult and infant diapers, training pants, sanitary napkins comprising an absorbent structure of the invention, the articles having an average caliper (thickness) in the crotch region of less than 1.0 cm, preferably less than 0.7cm, more preferably less than 0.5cm, or even less than 0.3cm (for this purpose alone, the crotch region being defined as the central zone of the product, when laid out flat and stretched, having a dimension of 20% of the length of the article and 50% of the width of the article).

Because the water-swellable material herein have a very good permeability, there is no need to have large amounts of traditional structuring agents presents, such as absorbent fibres, such as airfelt, and the may thus be omitted or only used in very small quantities, as described above. This further helps to reduce the thickness of the absorbent structure, or absorbent articles herein.

Preferred articles according to the present invention achieve a relatively narrow crotch width, which increases the wearing comfort. A preferred article according to the present invention achieves a crotch width of less than 100 mm, 90 mm, 80 mm, 70 mm, 60 mm or even less than 50 mm, as measured along a transversal line with is positioned at equal distance to the front edge and the rear edge of the article, or at the point with the narrowest transverse width. Hence, preferably an absorbent structure according to the present invention has a crotch width as measured along a transversal line with is positioned at equal distance to the front edge and the rear edge of the core which is of less than 100 mm, 90 mm, 80 mm, 70 mm, 60 mm or even less than 50 mm. It has been found that for most absorbent articles the liquid discharge occurs predominately in the front half.

A preferred diaper herein has a front waist band and a back waist band, whereby the front waist band and back waist band each have a first end portion and a second end portion and a middle portion located between the end portions, and whereby preferably the end portions comprise each a fastening system, to fasten the front waist band to the rear waist band or whereby preferably the end portions are connected to one another, and whereby the middle portion of the back waist band and/ or the back region of the backsheet and/or the crotch region of the backsheet comprises a landing member, preferably the landing member comprising second engaging elements selected from loops, hooks, slots, slits, buttons, magnets. Most preferred are hooks, adhesive or cohesive second engaging elements. Preferred may be that the engaging elements on the article, or preferably diaper are provided with a means to ensure they are only engage able at certain moments, for example, they may be covered by a removable tab, which is removed when the engaging elements are to be engaged and may be re-closed when engagement is no longer needed, as described above.

Preferred diapers and training pants herein have one or more sets of leg elastics and/or barrier leg cuffs, as known in the art.

Preferred may also be that the topsheet has an opening, preferably with elastication means along the length thereof, where through waste material can pass into a void space above the absorbent structure, and which ensures it is isolated in this void space, away from the wearer's skin.

Water-swellable material

The water-swellable material herein is such that it swells in water by absorbing the water; it may thereby form a gel. It may also absorb other liquids and swell. Thus, when used herein, 'water-swellable' means that the material swells at least in water, but typically also in other liquids or solutions, preferably in water based liquids such as 0.9% saline and urine.

The water-swellable material is solid; this includes gels, and particles, such as flakes, fibers, agglomerates, large blocks, granules, spheres, and other forms known in the art as 'solid' or 'particles'.

The water-swellable material herein comprises water-swellable particles containing water-swellable polymer(s) (particle), said water-swellable particles preferably being present at a level of at least 50% to 100% by weight (of the water-swellable material) or even from 80% to 100% by weight, and most preferably the material consists of said water-swellable particles. Said water-swellable particles of the water-swellable material preferably have a core-shell structure, as described herein, whereby the core preferably comprises said water-swellable polymer(s), which are typically also particulate.

The water-swellable material herein has an absorbent capacity of at least 20 g/g (as measured in the 4-hour CCRC test, described herein), preferably at least 25 g/g, or even more preferably at least 30 g/g, or even more preferably at least 40 g/g. The water swellable material herein may have an absorbent capacity of less than 80 g/g and or even less than 60 g/g as measured in the 4-hour CCRC test, described herein.

The water-swellable material herein has a Saline Absorbent Capacity (SAC), a Saline Absorbent Capacity after grinding (SAC") and a QUICS value calculated therefrom, as defined by the methods described hereinafter. The difference between SAC" and SAC and thus the QUICS calculated therefrom is a measure for the internal pressure exerted onto the core of the particles (containing water-swellable polymer) of the water-swellable material.

The QUICS values are as defined above, for the various water-swellable materials herein.

Highly preferred are water-swellable materials with a QUICS of at least 15, or more preferably at least 20, or even more preferably at least 30, and preferably up to 200 or even more preferably up to 150 or even more preferably up to 100.

The water-swellable material herein has a very high permeability or porosity, as represented by the CS-SFC value, as measured by the method set out herein.

The CS-SFC of the water-swellable material herein is typically at least $10 \times 10^{-7} cm^3 sec/g$, but preferably at least $30 \times 10^{-7} cm^3 sec/g$ or more preferably at least $50 \times 10^{-7} cm^3 sec/g$ or even more preferably at least $100 \times 10^{-7} cm^3 sec/g$. It may even be preferred that the CS-SFC is at least $500 \times 10^{-7} cm^3 sec/g$ or even more preferably at least $1000 \times 10^{-7} cm^3 sec/g$, and it has been found to be even possible to have a CS-SFC of $2000 \ 10^{-7} cm^3 sec/g$ or more.

Typically, the water-swellable material is particulate, having preferably particle sizes and distributions, which are about equal to the preferred particle sizes/distributions of the water-swellable polymer particles, as described herein below, even when these particles comprise a shell of for example elastomeric polymers, because this shell is typically very thin and does not significantly impact the particle size of the particles of the water-swellable material.

Surprisingly it has been found that, in contrast to water-swellable polymer particles known in the art, the particles of the water-swellable material herein are typically substantially spherical when swollen, for example when swollen by the method set out in the 4 hour CCRC test, described below. Namely, the particles are, even when swollen, able to withstand the average external pressure to such a degree that hardly any deformation of the particles takes place, ensuring the highly improved permeability.

The sphericity of the swollen particles can be determined (visualized) by for example the PartAn method or preferably by microscopy.

Preferably, the water-swellable material herein comprises elastomeric polymers, preferably present in or as a shell on the particle cores present in said material. The water absorbent materials herein have a surprisingly beneficial combination or balance of absorbent capacity, as measured in the 4 hour CCRC test and permeability, as measured in the CS-SFC test, set out herein.

In particular, the water-swellable materials herein have a particularly beneficial absorbency-distribution-index (ADI) of more than 1, preferably at least 2, more preferably at least 3, even more preferably at least 6 and most preferable of at least about 10, whereby the ADI is defined as:

$$ADI = (CS\text{-}SFC^1 / (150 * 10^{-7} cm^3 sec/g)) / I_0{}^{2\text{-}5\text{-}0\text{-}095} * {}^{(CS\text{-}CC_R{}^{C/g/g)}}$$

CS-CCRC is the Cylinder Centrifuge Retention Capacity after 4 hours of swelling as set out in the test method section below.

Typically, the water-swellable materials will have an ADI of not more than about 200 and preferably not more than 50.

Shells and preferred elastomeric polymers thereof

The water-swellable material herein comprises water-swellable particles, with a core-shell structure. Said core comprises water- swellable polymer(s). Said shell (on said core) comprises elastomeric polymers.

For the purpose of the invention, it should be understood that the shell will be present on at least a portion of the surface of the core, referred to herein; this includes the embodiment that said shell may form the outer surface of the particles, and the embodiment that the shell does not form the outer surface of the particles.

In a preferred execution, the water-swellable material comprises, or consists of, water-swellable particles, which have a core formed by particulate water-swellable polymer(s), as described herein, and this core forms the centre of the particles of the water-swellable material herein, and the water-swellable particles comprise each a shell, which is present on substantially the whole outer surface area of said core.

In one preferred embodiment herein, the shell is an essentially continuous layer around the water-swellable polymer core, and said layer covers the entire surface of the polymer core, i.e., no regions of the core surface are exposed. Hereto, the shell is typically formed by the preferred processes described herein after.

The shell, preferably formed in the preferred process described herein, is preferably pathwise connected and more preferably, the shell is pathwise connected and encapsulating (completely circumscribing) the core, e.g., of water-swellable polymer(s) (see, for example, E. W. Weinstein et. al, Mathworld - A Wolfram Web Resource for 'encapsulation' and 'pathwise connected'). The shell is preferably a pathwise connected complete surface on the surface of the core. This complete surface consists of first areas where the shell is present and which are pathwise connected, e.g., like a network, but it may comprise second areas, where no shell is present, being for example micro pores, whereby said second areas are a disjoint union. Preferably, each second area, e.g., micropore, has a surface area of less than 0.1 $mm^2$, or even less than $0.01mm^2$ preferably less than 8000 $\mu m^2$, more preferably less than 2000 $\mu m^2$ and even more preferably less than $80\mu m^2$. However, it is most preferred that no second areas are present, and that the shell forms a complete encapsulation around the core, e.g., of water-swellable polymer(s).

As said above, the shell preferably comprises elastomeric polymers, as described hereinafter. The shell of elastomeric polymers is preferably formed on the surface of the core of water-swellable polymer(s) by the method described hereinafter, a dispersion or solution of the elastomeric polymers is sprayed onto the core of water- swellable polymers by the preferred processes described herein. It has surprisingly been found that these preferred process conditions further improve the resistance of the shell against pressure, improving the permeability of the water-swellable material whilst ensuring a good absorbency.

The shells herein have in general a high shell tension, which is defined as the (Theoretical equivalent shell caliper) x (Average wet secant elastic modulus at 400% elongation), of 5 to 200 N/m, or preferably of 10 to 170N/m, or more preferably 20 to 130 N/m. In some embodiments it may be preferred to have a shell with a shell tension of 40N/m to 110N/m.

In one embodiment herein, where the water-swellable polymers herein have been (surface) post-crosslinked (either prior to application of the shell described herein, or at the same time as applying said shell), it may even be more preferred that the shell tension is in the range from 15 N/m to 60N/m, or even more preferably from 20 N/m to 60N/m, or preferably from 40 to 60 N/m.

In yet another embodiment wherein the water-swellable polymers are not surface-crosslinked, it may even be more preferred that said shell tension is in the range from more than 60 N/m to 110 N/m.

The shell is preferably at least moderately water-permeable (breathable) with a moisture vapor transmission rate (MVTR; as can be determined by the method set out below) of more than 200 g/m²/day, preferably breathable with a MVTR of 800 g/m²/day or more preferably 1200 to (inclusive) 1400 g/m²/day, even more preferably breathable with a MVTR of at least 1500 g/m²/day, up to 2100 g/m²/day (inclusive), and most preferably the shell (e.g., the elastomeric polymer) is highly breathable with a MVTR of 2100 g/m²/day or more.

The shell herein is typically thin; preferably the shell has an average caliper (thickness) between 1 micron ($\mu$m) and 100 microns, preferably from 1 micron to 50 microns, more preferably from 1 micron to 20 microns or even from 2 to 20 microns or even from 2 to 10 microns, as can be determined by the method described herein.

The shell is preferably uniform in caliper and/or shape. Preferably, the average caliper is such that the ratio of the smallest to largest caliper is from 1:1 to 1:5, preferably from 1:1 to 1:3, or even 1:1 to 1:2, or even 1:1 to 1:1.5.

Preferably, the water-swellable material has a shell of elastomeric polymer(s), which are typically film-forming elastomeric polymers, and typically thermoplastic film-forming elastomeric polymers.

The elastomeric polymers herein are non water-swellable. They typically absorb less than 1.0 g/g water or saline or synthetic urine, preferably even less than 0.5 g/g, or even less than 0.1 g/g, as may be determined by the method described herein.

The elastomeric polymer may be a polymer with at least one glass transition temperature of below 60°C; preferred may be that the elastomeric polymer is a block copolymer, whereby at least one segment or block of the copolymer has a Tg below room temperature (i.e., below 25°C; this is said to be the soft segment or soft block) and at least one segment or block of the copolymer that has a Tg above room temperature (and this is said to be the hard segment or hard block)., as described in more detail below. The Tg's, as referred to herein, may be measured by methods known in the art, such as Differential Scanning Calorimetry (DSC) to measure the change in specific heat that an elastomeric polymer material undergoes upon heating. The DSC measures the energy required to maintain the temperature of a sample of the elastomeric polymer to be the same as the temperature of the inert reference material (e.g., Indium). A Tg is determined from the midpoint of the endothermic change in the slope of the baseline. The Tg values are reported from the second heating cycle so that any residual solvent in the sample is removed.

Preferably, the water-swellable material comprises particles with a shell that comprises one or more elastomeric polymers (with at least one Tg of less than 60°C and said material has a shell impact parameter, which is defined as the (Average wet secant elastic modulus at 400% elongation) * (Relative Weight of said elastomeric polymer compared to the total weight of the water-swellable material) of 0.03 MPa to 0.6 MPa, preferably 0.07 MPa to 0.45 MPa, more preferably of 0.1 to 0.35 MPa.

The relative weight percentage of the elastomeric polymer above may be determined by for example the pulsed NMR method described herein.

In a preferred embodiment, the water-swellable material comprises elastomeric polymers, typically present in the shell of the particles thereof, which are typically present at a weight percentage of (by weight of the water-swellable material) of 0.1% to 25%, or more preferably 0.5 to 15% or even more preferably to 10%, or even more preferably up to 5%. The skilled person would know the suitable methods to determine this. For example, for water-swellable materials comprising elastomeric polymers with at least one glass transition temperature (Tg) of less than 60°C or less, the NMR method described herein below may be used.

In order to impart desirable properties to the elastomeric polymer, additionally fillers such as particulates, oils, solvents, plasticizers, surfactants, dispersants may be optionally incorporated.

The elastomeric polymer may be hydrophobic or hydrophilic. For fast wetting it is, however, preferable that the polymer is also hydrophilic.

The elastomeric polymer is applied as, and present as in the form of a shell on the water-swellable polymer particles, and this is done by coating processes described herein, by use of a solution or a dispersion thereof. Such solutions and dispersions can be prepared using water and/ or any suitable organic solvent, for example acetone, isopropanol, tetrahydrofuran, methyl ethyl ketone, dimethyl sulfoxide, dimethylformamide, chloroform, ethanol, methanol and mixtures thereof.

In a preferred embodiment the polymer is applied in the form of a, preferably aqueous, dispersion and in a more preferred embodiment the polymer is applied as an aqueous dispersion of a polyurethane, such as the preferred polyurethanes described below.

The synthesis of polyurethanes and the preparation of polyurethane dispersions is well described for example in Ullmann's Encyclopedia of Industrial Chemistry, Sixth Edition, 2000 Electronic Release.

The polyurethane is preferably hydrophilic and in particular surface hydrophilic. The surface hydrophilicity may be determined by methods known to those skilled in the art. In a preferred execution, the hydrophilic polyurethanes are materials that are wetted by the liquid that is to be absorbed (0.9% saline; urine). They may be characterized by a contact angle that is less than 90 degrees. Contact angles can for example be measured with the Video-based contact angle measurement device, Krüss GIO - G1041, available from Kruess, Germany or by other methods known in the art.

In a preferred embodiment, the hydrophilic properties are achieved as a result of the polyurethane comprising hydrophilic polymer blocks, for example polyether groups having a fraction of groups derived from ethylene glycol $(CH_2CH_2O)$ or from 1,4-butanediol $(CH_2CH_2CH_2CH_2O)$ or from propylene glycol $(CH_2CH_2CH_2O)$, or mixtures thereof.

Polyetherpolyurethanes are therefore the elastomeric polymers. The hydrophilic blocks can be constructed in the manner of comb polymers where parts of the side chains or all side chains are hydrophilic polymeric blocks. But the hydrophilic blocks can also be constituents of the main chain (i.e., of the polymer's backbone). A preferred embodiment utilizes polyurethanes where at least the predominant fraction of the hydrophilic polymeric blocks is present in the form of side chains. The side chains can in turn be block copolymers such as poly(ethylene glycol)-co-poly(propylene glycol).

Highly preferred are polyetherpolyurethanes with side chains with alkylene oxide units, preferably ethylene oxide units. Also preferred are polyetherpolyurethanes whereby the main chain comprises alkylene oxide units, preferably butylene oxide units.

It is further possible to obtain hydrophilic properties for the polyurethanes through an elevated fraction of ionic groups, preferably carboxylate, sulfonate, phosphonate or ammonium groups. The ammonium groups may be protonated or alkylated tertiary or quarternary groups. Carboxylates, sulfonates, and phosphates may be present as alkalimetal or ammonium salts. Suitable ionic groups and their respective precursors are, for example, described in "Ullmanns Encyclopadie der technischen Chemie", 4th Edition, Volume 19, p. 311-313 and are furthermore described in DE-A 1 495 745 and WO 03/050156.

The hydrophilicity of the preferred polyurethanes facilitates the penetration and dissolution of water into the water-swellable polymeric particles which are enveloped by the elastomeric polymer (shell).

Especially preferred polyurethanes herein comprise one or more phase-separating block copolymers, having a weight average molecular weight Mw of at least 5 kg/mol, preferably at least 10 kg/mol and higher.

In one embodiment such a block copolymer has at least a first polymerized homopolymer segment (block) and a second polymerized homopolymer segment (block), polymerized with one another, whereby preferably the first (soft) segment has a $Tg_1$ of less than 20°C, or even less than 0°C, and the second (hard) segment has a $Tg_2$ of preferably 60°C or more or even 70°C or more.

In another embodiment, such a block copolymer has at least a first polymerized heteropolymer segment (block) and a second polymerized heteropolymer segment (block), polymerized with one another, whereby preferably the first (soft) segment has a $Tg_1$ of less than 20°C, or even less than 0°C, and the second (hard) segment has a $Tg_2$ of preferably 60°C or more or even 70°C or more.

In one embodiment the total weight average molecular weight of the hard second segments (with a Tg of at least 50°C) is preferably at least 28 kg/mol, or even at least 45 kg/mol.

The preferred weight average molecular weight of a first (soft) segment (with a Tg of less than 20°C) is at least 500 g/mol, preferably at least 1000 g/mol or even at least 2000 g/mol, but preferably less than 8000 g/mol, preferably less than 5000 g/mol.

However, the total of the first (soft) segments is typically 20% to 95% by weight of the total block copolymer, or even from 20% to 85% or more preferably from 30% to 75% or even from 40% to 70% by weight. Furthermore, when the total weight level of soft segments is more than 70%, it is even more preferred that an individual soft segment has a weight average molecular weight of less than 5000 g/mol.

It is well understood by those skilled in the art that "polyurethanes" is a generic term used to describe polymers that are obtained by reacting di- or polyisocyanates with at least one di- or polyfunctional "active hydrogen-containing" compound. "Active hydrogen containing" means that the di- or polyfunctional compound has at least 2 functional groups which are reactive toward isocyanate groups (also referred to as reactive groups), e.g., hydroxyl groups, primary and secondary amino groups and mercapto (SH) groups.

It also is well understood by those skilled in the art that polyurethanes also include allophanate, biuret, carbodiimide, oxazolidinyl, isocyanurate, uretdione, and other linkages in addition to urethane and urea linkages.

In one embodiment the block copolymers useful herein are preferably polyether urethanes and polyester urethanes. Especially preferred are polyether urethanes comprising polyalkylene glycol units, especially polyethylene glycol units or poly(tetramethylene glycol) units.

As used herein, the term "alkylene glycol" includes both alkylene glycols and substituted alkylene glycols having 2 to 10 carbon atoms, such as ethylene glycol, 1,3-propylene glycol, 1,2-propylene glycol, 1,2-butylene glycol, 1,3-butylene glycol, 1,4-butylene glycol, styrene glycol and the like.

The polyurethanes used herein are generally obtained by reaction of polyisocyanates with active hydrogen-containing compounds having two or more reactive groups. These include:

a) high molecular weight compounds having a molecular weight in the range of preferably 300 to 100 000 g/mol especially from 500 to 30 000 g/mol;
b) low molecular weight compounds; and
c) compounds having polyether groups, especially polyethylene oxide groups or polytetrahydrofuran groups and a molecular weight in the range from 200 to 20 000 g/mol, the polyether groups in turn having no reactive groups.

These compounds can also be used as mixtures.

Suitable polyisocyanates have an average of about two or more isocyanate groups, preferably an average of about two to about four isocyanate groups and include aliphatic, cycloaliphatic, araliphatic, and aromatic polyisocyanates, used alone or in mixtures of two or more. Diisocyanates are more preferred. Especially preferred are aliphatic and cycloaliphatic polyisocyanates, especially diisocyanates.

Specific examples of suitable aliphatic diisocyanates include alpha, omega-alkylene diisocyanates having from 5 to 20 carbon atoms, such as hexamethylene-1,6-diisocyanate, 1,12-dodecane diisocyanate, 2,2,4-trimethylhexamethylene diisocyanate, 2,4,4-trimethyl-hexamethylene diisocyanate, 2-methyl-1,5-pentamethylene diisocyanate, and the like. Polyisocyanates having fewer than 5 carbon atoms can be used but are less preferred because of their high volatility and toxicity. Preferred aliphatic polyisocyanates include hexamethylene-1,6-diisocyanate, 2,2,4-trimethyl-hexamethylene diisocyanate, and 2,4,4-trimethyl-hexamethylene diisocyanate.

Specific examples of suitable cycloaliphatic diisocyanates include dicyclohexylmethane diisocyanate, (commercially available as Desmodur® W from Bayer Corporation), isophorone diisocyanate, 1,4-cyclohexane diisocyanate, 1,3-bis(isocyanatomethyl) cyclohexane, and the like. Preferred cycloaliphatic diisocyanates include dicyclohexylmethane diisocyanate and isophorone diisocyanate.

Specific examples of suitable araliphatic diisocyanates include m-tetramethyl xylylene diisocyanate, p-tetramethyl xylylene diisocyanate, 1,4-xylylene diisocyanate, 1,3-xylylene diisocyanate, and the like. A preferred araliphatic diisocyanate is tetramethyl xylylene diisocyanate.

Examples of suitable aromatic diisocyanates include 4,4'-diphenylmethane diisocyanate, toluene diisocyanate, their isomers, naphthalene diisocyanate, and the like. A preferred aromatic diisocyanate is toluene diisocyanate and 4,4'-diphenylmethane diisocyanate.

Examples of high molecular weight compounds a) having 2 or more reactive groups are such as polyester polyols and polyether polyols, as well as polyhydroxy polyester amides, hydroxyl-containing polycaprolactones, hydroxyl-containing acrylic copolymers, hydroxyl-containing epoxides, polyhydroxy polycarbonates, polyhydroxy polyacetals, polyhydroxy polythioethers, polysiloxane polyols, ethoxylated polysiloxane polyols, polybutadiene polyols and hydrogenated polybutadiene polyols, polyacrylate polyols, halogenated polyesters and polyethers, and the like, and mixtures thereof. The polyester polyols, polyether polyols, polycarbonate polyols, polysiloxane polyols, and ethoxylated polysiloxane polyols are preferred. Particular preference is given to polyesterpolyols, polycarbonate polyols and polyalkylene ether polyols. The number of functional groups in the aforementioned high molecular weight compounds is preferably on average in the range from 1.8 to 3 and especially in the range from 2 to 2.2 functional groups per molecule.

The polyester polyols typically are esterification products prepared by the reaction of organic polycarboxylic acids or their anhydrides with a stoichiometric excess of a diol.

The diols used in making the polyester polyols include alkylene glycols, e.g., ethylene glycol, 1,2- and 1,3-propylene glycols, 1,2-, 1,3-, 1,4-, and 2,3-butane diols, hexane diols, neopentyl glycol, 1,6-hexanediol, 1,8-octanediol, and other glycols such as bisphenol-A, cyclohexanediol, cyclohexane dimethanol (1,4-bis-hydroxymethylcyclo-hexane), 2-methyl-1,3-propanediol, 2,2,4-trimethyl-1,3-pentanediol, diethylene glycol, triethylene glycol, tetraethylene glycol, polyethylene glycol, dipropylene glycol, polypropylene glycol, dibutylene glycol, polybutylene glycol, dimerate diol, hydroxylated bisphenols, polyether glycols, halogenated diols, and the like, and mixtures thereof. Preferred diols include ethylene glycol, diethylene glycol, butane diol, hexane diol, and neopentylglycol. Alternatively or in addition, the equivalent mercapto compounds may also be used.

Suitable carboxylic acids used in making the polyester polyols include dicarboxylic acids and tricarboxylic acids and anhydrides, e.g., maleic acid, maleic anhydride, succinic acid, glutaric acid, glutaric anhydride, adipic acid, suberic acid, pimelic acid, azelaic acid, sebacic acid, chlorendic acid, 1,2,4-butane-tricarboxylic acid, phthalic acid, the isomers of phthalic acid, phthalic anhydride, fumaric acid, dimeric fatty acids such as oleic acid, and the like, and mixtures thereof. Preferred polycarboxylic acids used in making the polyester polyols include aliphatic or aromatic dibasic acids.

Examples of suitable polyester polyols include poly(glycol adipate)s, poly(ethylene terephthalate) polyols, polycaprolactone polyols, orthophthalic polyols, sulfonated and phosphonated polyols, and the like, and mixtures thereof.

The preferred polyester polyol is a diol. Preferred polyester diols include poly(butanediol adipate); hexanediol adipic acid and isophthalic acid polyesters such as hexaneadipate isophthalate polyester; hexanediol neopentyl glycol adipic acid polyester diols, e.g., Piothane 67-3000 HNA (Panolam Industries) and Piothane 67-1000 HNA, as well as propylene glycol maleic anhydride adipic acid polyester diols, e.g., Piothane SO-1000 PMA, and hexane diol neopentyl glycol fumaric acid polyester diols, e.g., Piothane 67-SO0 HNF. Other preferred Polyester diols include Rucoflex® S101.5-3.5, S1040-3.5, and S-1040-110 (Bayer Corporation).

Polyether polyols are obtained in known manner by the reaction of a starting compound that contains reactive hydrogen atoms, such as water or the diols set forth for preparing the polyester polyols, and alkylene glycols or cyclic ethers, such as ethylene glycol, propylene glycol, butylene glycol, styrene glycol, ethylene oxide, propylene oxide, 1,2-butylene oxide, 2,3-butylene oxide, oxetane, tetrahydrofuran, epichlorohydrin, and the like, and mixtures thereof. Preferred polyethers include poly(ethylene glycol), poly(propylene glycol), polytetrahydrofuran, and co [poly(ethylene glycol)-poly(propylene glycol)]. Polyethylenglycol and Polypropyleneglycol can be used as such or as physical blends. In case that propyleneoxide and ethylenoxide are copolymerized, these polypropylene-co-polyethylene polymers can be used as random polymers or blockcopolymers.

In one embodiment the polyetherpolyol is a constituent of the main polymer chain.

In another embodiment the polyetherol is a terminal group of the main polymer chain.

In yet another embodiment the polyetherpolyol is a constituent of a side chain which is comb-like attached to the main chain. An example of such a monomer is Tegomer D-3403 (Degussa).

Polycarbonates include those obtained from the reaction of diols such 1,3-propanediol, 1,4-butanediol, 1,6-hexanediol, diethylene glycol, triethylene glycol, tetraethylene glycol, and the like, and mixtures thereof with dialkyl carbonates such as diethyl carbonate, diaryl carbonates such as diphenyl carbonate or phosgene.

Examples of low molecular weight compounds b) having two reactive functional groups are the diols such as alkylene glycols and other diols mentioned above in connection with the preparation of polyesterpolyols. They also include amines such as diamines and polyamines which are among the preferred compounds useful in preparing the aforesaid polyesteramides and polyamides. Suitable diamines and polyamines include 1,2-diaminoethane, 1,6-diaminohexane, 2-methyl-1,5-pentanediamine, 2,2,4-trimethyl-1,6-hexanediamine, 1,12-diaminododecane, 2-aminoethanol, 2-[(2-aminoethyl)amino]-ethanol, piperazine, 2,5-dimethylpiperazine, 1-amino-3-aminomethyl-3,5,5-trimethylcyclohexane (isophorone diamine or IPDA), bis-(4-aminocyclohexyl)-methane, bis-(4-amino-3-methyl-cyclohexyl)-methane, 1,4-diaminocyclohexane, 1,2-propylenediamine, hydrazine, urea, amino acid hydrazides, hydrazides of semicarbazidocarboxylic acids, bis-hydrazides and bis-semicarbazides, diethylene triamine, triethylene tetramine, tetraethylene pentamine, pentaethylene hexamine, N,N,N-tris-(2-aminoethyl)amine, N-(2-piperazinoethyl)-ethylene diamine, N,N'-bis-(2-aminoethyl)-piperazine, N,N,N'-tris-(2-aminoethyl)ethylene diamine, N-[N-(2-aminoethyl)-2-aminoethyl]-N'-(2-aminoethyl)-piperazine, N-(2-aminoethy)-N'-(2-piperazinoethyl)-ethylene diamine, N,N-bis-(2-aminoethyl)-N-(2-piperazinoethyl)amine, N,N-bis-(2-piperazinoethyl)amine, polyethylene imines, iminobispropylamine, guanidine, melamine, N-(2-aminoethyl)-1,3-propane diamine, 3,3'-diaminobenzidine, 2,4,6-triaminopyrimidine, polyoxypropylene amines, tetrapropylenepentamine, tripropylenetetramine, N,N-bis-(6-aminohexyl)amine, N,N'-bis-(3-aminopropyl)ethylene diamine, and 2,4-bis-(4'-aminobenzyl)-aniline, and the like, and mixtures thereof. Preferred diamines and polyamines include 1-amino-3-aminomethyl-3,5,5-trimethylcyclohexane (isophorone diamine or IPDA), bis-(4-aminocyclohexyl)-methane, bis-(4-amino-3-methylcyclohexyl)-methane, ethylene diamine, diethylene triamine, triethylene tetramine, tetraethylene pentamine, and pentaethylene hexamine, and the like, and mixtures thereof. Other suitable diamines and polyamines for example include Jeffamine® D-2000 and D-4000, which are amine-terminated polypropylene glycols differing only by molecular weight, and Jeffamine® XTJ-502, T 403, T 5000, and T 3000 which are amine terminated polyethyleneglycols, amine terminated co-polypropylene-polyethylene glycols, and triamines based on propoxylated glycerol or trimethylolpropane and which are available from Huntsman Chemical Company.

The poly(alkylene glycol) may be part of the polymer main chain or be attached to the main chain in comb-like shape as a side chain.

In a preferred embodiment, the polyurethane comprises poly(alkylene glycol) side chains sufficient in amount to comprise about 10 wt.% to 90 wt.%, preferably about 12 wt.% to about 80 wt.%, preferably about 15 wt.% to about 60 wt.%, and more preferably about 20 wt.% to about 50 wt.%, of poly(alkylene glycol) units in the final polyurethane on a dry weight basis. At least about 50 wt.%, preferably at least about 70 wt.%, and more preferably at least about 90 wt.% of the poly(alkylene glycol) side-chain units comprise poly(ethylene glycol), and the remainder of the side-chain poly-(alkylene glycol) units can comprise alkylene glycol and substituted alkylene glycol units having from 3 to about 10 carbon atoms. The term "final polyurethane" means the polyurethane used for the shell of the water- polymeric particles.

Preferably the amount of the side-chain units is (i) at least about 30 wt.% when the molecular weight of the side-chain units is less than about 600 g/mol, (ii) at least about 15 wt.% when the molecular weight of the side-chain units is from about 600 to about 1000 g/mol, and (iii) at least about 12 wt.% when the molecular weight of said side-chain units is more than about 1000 g/mol. Mixtures of active hydrogen-containing compounds having such poly(alkylene glycol) side chains can be used with active hydrogen-containing compounds not having such side chains.

These side chains can be incorporated in the polyurethane by replacing a part or all of the aforementioned high molecular diols a) or low molecular compounds b) by compounds c) having at least two reactive functional groups and a polyether group, preferably a polyalkylene ether group, more preferably a polyethylene glycol group that has no further reactive group.

For example, active hydrogen-containing compounds having a polyether group, in particular a poly(alkylene glycol) group, include diols having poly(ethylene glycol) groups such as those described in U.S. Pat. No. 3,905,929 (incorporated herein by reference in its entirety). Further, U.S. Pat. No. 5,700,867 (incorporated herein by reference in its entirety) teaches methods for incorporation of poly(ethylene glycol) side chains at col. 4, line 3.5 to col. 5, line 4.5. A preferred active hydrogen-containing compound having poly(ethylene glycol) side chains is trimethylol propane mono (polyethylene oxide methyl ether), available as Tegomer D-3403 from Degussa-Goldschmidt.

Preferably, the polyurethanes to be used in the present invention also have reacted therein at least one active hydrogen-containing compound not having said side chains and typically ranging widely in molecular weight from about 50 to about 10,000 g/mol, preferably about 200 to about 6000 g/mol, and more preferably about 300 to about 3000 g/mol. Suitable active hydrogen-containing compounds not having said side chains include any of the amines and polyols described herein as compounds a) and b).

According to one preferred embodiment of the invention, the active hydrogen compounds are chosen to provide less than about 25 wt.%, more preferably less than about 15 wt.% and most preferably less than about 5 wt.% poly(ethylene glycol) units in the backbone (main chain) based upon the dry weight of final polyurethane, since such main-chain poly(ethylene glycol) units tend to cause swelling of polyurethane particles in the waterborne polyurethane dispersion and also contribute to lower in use tensile strength of articles made from the polyurethane dispersion.

The preparation of polyurethanes having polyether side chains is known to one skilled in the art and is extensively described for example in US 2003/0195293.

The present invention accordingly also provides absorbent structures with a water-swellable material comprising water-swellable polymeric particles with an elastomeric polyurethane shell, wherein the polyurethane comprises not only side chains having polyethylene oxide units but also polyethylene oxide units in the main chain.

Advantageous polyurethanes are obtained by first preparing prepolymers having isocyanate end groups, which are subsequently linked together in a chain-extending step. The linking together can be through water or through reaction with a compound having at least one crosslinkable functional group.

The prepolymer is obtained by reacting one of the above-described isocyanate compounds with an active hydrogen compound. Preferably the prepolymer is prepared from the above mentioned polyisocyanates, at least one compound c) and optionally at least one further active hydrogen compound selected from the compounds a) and b).

In one embodiment the ratio of isocyanate to active hydrogen in the compounds forming the prepolymer typically ranges from about 1.3/1 to about 2.5/1, preferably from about 1.5/1 to about 2.1/1, and more preferably from about 1.7/1 to about 2/1.

The polyurethane may additionally contain functional groups which can undergo further crosslinking reactions and which can optionally render them self-crosslinkable.

Compounds having at least one additional crosslinkable functional group include those having carboxylic, carbonyl, amine, hydroxyl, and hydrazide groups, and the like, and mixtures of such groups. The typical amount of such optional compound is up to about 1 milliequivalent, preferably from about 0.05 to about 0.5 milliequivalent, and more preferably from about 0.1 to about 0.3 milliequivalent per gram of final polyurethane on a dry weight basis.

The preferred monomers for incorporation into the isocyanate-terminated prepolymer are hydroxy-carboxylic acids having the general formula $(HO)_xQ(COOH)y$ wherein Q is a straight or branched hydrocarbon radical having 1 to 12 carbon atoms, and x and y are 1 to 3. Examples of such hydroxy-carboxylic acids include citric acid, dimethylolpropanoic acid (DMPA), dimethylol butanoic acid (DMBA), glycolic acid, lactic acid, malic acid, dihydroxymalic acid, tartaric acid, hydroxypivalic acid, and the like, and mixtures thereof. Dihydroxy-carboxylic acids are more preferred with dimethylolpropanoic acid (DMPA) being most preferred.

Other suitable compounds providing crosslinkability include thioglycolic acid, 2,6-dihydroxybenzoic acid, and the like, and mixtures thereof.

Optional neutralization of the prepolymer that has pendant carboxyl groups converts the carboxyl groups to carboxylate anions, thus having a water-dispersibility enhancing effect. Suitable neutralizing agents include tertiary amines, metal hydroxides, ammonia, and other agents well known to those skilled in the art.

As a chain extender, at least one of water, an inorganic or organic polyamine having an average of about 2 or more primary and/or secondary amine groups, polyalcohols, ureas, or combinations thereof is suitable herein. Suitable organic amines for use as a chain extender include diethylene triamine (DETA), ethylene diamine (EDA), meta-xylylenediamine (MXDA), aminoethyl ethanolamine (AEEA), 2-methyl pentane diamine, and the like, and mixtures thereof. Also suitable herein are propylene diamine, butylene diamine, hexamethylene diamine, cyclohexylene diamine, phenylene diamine, tolylene diamine, 3,3-dichlorobenzidene, 4,4'-methylene-bis-(2-chloroaniline), 3,3-dichloro- 4,4-diamino diphenylmethane, sulfonated primary and/or secondary amines, and the like, and mixtures thereof. Suitable inorganic and organic amines include hydrazine, substituted hydrazines, and hydrazine reaction products, and the like, and mixtures thereof. Suitable polyalcohols include those having from 2 to 12 carbon atoms, preferably from 2 to 8 carbon atoms, such as ethylene glycol, diethylene glycol, neopentyl glycol, butanediols, hexanediol, and the like, and mixtures thereof. Suitable ureas include urea and its derivatives, and the like, and mixtures thereof. Hydrazine is preferred and is most preferably used as a solution in water. The amount of chain extender typically ranges from about 0.5 to about 0.95 equivalents based on available isocyanate.

A degree of branching of the polyurethane may be beneficial, but is not required, to maintain a high tensile strength and improve resistance to creep (cf. strain relaxation). This degree of branching may be accomplished during the prepolymer step or the extension step. For branching during the extension step, the chain extender DETA is preferred, but other amines having an average of about two or more primary and/or secondary amine groups may also be used. For branching during the prepolymer step, it is preferred that trimethylol propane (TMP) and other polyols having an average of more than two hydroxyl groups be used. The branching monomers can be present in amounts up to about 4 wt. % of the polymer backbone.

Polyurethanes are preferred elastomeric polymers. They can be applied to the water-swellable polymer particles from solvent or from a dispersion. Particularly preferred are aqueous dispersions.

Preferred aqueous polyurethane dispersions are Hauthane HD-4638 (ex Hauthaway), Hydrolar HC 269 (ex Colm, Italy), Impraperm 48180 (ex Bayer Material Science AG, Germany), Lupraprot DPS (ex BASF Germany), Permax 120, Permax 200, and Permax 220 (ex Noveon, Brecksville, OH), ), Syntegra YM2000 and Syntegra YM2100 (ex Dow, Midland, Michigan) Witcobond G-213, Witcobond G-506, Witcobond G-507, and Witcobond 736 (ex Uniroyal Chemical, Middlebury, CT).

Particularly suitable elastomeric polyurethanes are extensively described in the literature references hereinbelow and expressly form part of the subject matter of the present disclosure. Particularly hydrophilic thermoplastic polyurethanes are sold by Noveon, Brecksville, Ohio, under the tradenames of Permax 120, Permax 200 and Permax 220 and are described in detail in "Proceedings International Waterborne High Solids Coatings, 32, 299, 2004" and were presented to the public in February 2004 at the "International Waterborne, High-Solids, and Powder Coatings Symposium" in New Orleans, USA. The preparation is described in detail in US 2003/0195293.

Furthermore, the polyurethanes described in US 4,190,566, US 4,092,286, US 2004/0214937 and also WO 03/050156.

More particularly, the polyurethanes described can be used in mixtures with each other or with other elastomeric polymers, fillers, oils, water-soluble polymers or plasticizing agents in order that particularly advantageous properties may be achieved with regard to hydrophilicity, water perviousness and mechanical properties.

It may be preferred that the elastomeric polymers herein comprises fillers to reduce tack such as the commercially available resin Estane 58245-047P and Estane X-1007-040P, available from Noveon Inc., 9911 Brecksville Road, Cleveland, OH 44141-3247, USA.

Alternatively such fillers can be added in order to reduce tack to the dispersions or solutions of suitable elastomeric polymers before application. A typical filler is Aerosil, but other inorganic deagglomeration aids as listed below can also be used.

Preferred polyurethanes for use herein are strain hardening and/or strain crystallizing. Strain Hardening is observed during stress-strain measurements, and is evidenced as the rapid increase in stress with increasing strain. It is generally believed that strain hardening is caused by orientation of the polymer chains in the film producing greater resistance to extension in the direction of drawing.

Water-swellable polymers

The water-swellable polymers herein are preferably solid, preferably in the form of particles (which includes, for example, particles in the form of flakes, fibers, agglomerates). The water-swellable polymer particles can be spherical in shape as well as irregularly shaped particles.

Useful herein are in principle all particulate water-swellable polymers known to one skilled in the art from superabsorbent literature for example as described in Modern Superabsorbent Polymer Technology, F.L. Buchholz, A.T. Graham, Wiley 1998. The water-swellable particles are preferably spherical water-swellable particles of the kind typically obtained from inverse phase suspension polymerizations; they can also be optionally agglomerated at least to some extent to form larger irregular particles. But most particular preference is given to commercially available irregularly shaped particles of the kind obtainable by current state of the art production processes as is more particularly described herein below by way of example.

Olefinically unsaturated carboxylic acid and anhydride monomers useful herein include the acrylic acids typified by acrylic acid itself, methacrylic acid, α-chloroacrylic acid, α-cyanoacrylic acid, β-methylacrylic acid (crotonic acid), α-phenylacrylic acid, β-acryloxypropionic acid, sorbic acid, α-chlorosorbic acid, angelic acid, cinnamic acid, p-chlorocinnamic acid, β-stearylacrylic acid, itaconic acid, citroconic acid, mesaconic acid, glutaconic acid, aconitic acid, maleic acid, fumaric acid, tricarboxyethylene, and maleic anhydride. Preferred water-swellable polymers contain carboxyl groups, such as the above-described carboxylic acid/carboxylate containing groups. These polymers include hydrolyzed starch-acrylonitrile graft copolymers, partially neutralized hydrolyzed starch-acrylonitrile graft copolymers, starch-acrylic acid graft copolymers, partially neutralized starch-acrylic acid graft copolymers, hydrolyzed vinyl acetate-acrylic ester copolymers, hydrolyzed acrylonitrile or acrylamide copolymers, slightly network crosslinked polymers of any of the aforementioned copolymers, polyacrylic acid, and slightly network crosslinked polymers of polyacrylic acid.

The water-swellable polymers are preferably polymeric particles obtainable by polymerization of a monomer solution comprising:

> i) at least one ethylenically unsaturated acid-functional monomer,
> ii) at least one crosslinker,
> iii) if appropriate one or more ethylenically and/or allylically unsaturated monomers copolymerizable with i), and
> iv) if appropriate one or more water-soluble polymers onto which the monomers i), ii) and if appropriate iii) can be at least partially grafted,
> wherein the base polymer obtained thereby is dried, classified and if appropriate is subsequently treated with
> v) at least one post-crosslinker (or: surface cross-linker)

before being dried and optionally post-crosslinked (i.e., Surface crosslinked).

Useful monomers i) include, for example, ethylenically unsaturated carboxylic acids, such as acrylic acid, methacrylic acid, maleic acid, fumaric acid, and itaconic acid, or derivatives thereof, such as acrylamide, methacrylamide, acrylic esters and methacrylic esters. Acrylic acid and methacrylic acid are particularly preferred monomers.

The water-swellable polymers to be used herein are typically crosslinked, i.e., the polymerization is carried out in the presence of compounds having two or more polymerizable groups which can be free-radically copolymerized into the polymer network.

The preparation of a suitable base polymer and also further useful hydrophilic ethylenically unsaturated monomers i) are described in DE-A 199 41 423, EP-A 686 650, WO 01/45758 and WO 03/14300.

The acid groups of the base polymers obtained are preferably 30 - 100 mol%, more preferably 65 - 90 mol% and most preferably 72 - 85 mol% neutralized, for which the customary neutralizing agents can be used.

Neutralization can be carried out after polymerization, at the base polymer stage. But it is also possible to neutralize up to 40 mol%, preferably from 10 to 30 mol% and more preferably from 15 to 25 mol% of the acid groups before polymerization by adding a portion of the neutralizing agent to the monomer solution and to set the desired final degree of neutralization only after polymerization, at the base polymer stage.

Most preferably, the water-swellable polymers comprise from about 50% to 95% (mol percentage), preferably about 75 mol% neutralized, (slightly) crosslinked, polyacrylic acid (i.e., poly (sodium acrylate/acrylic acid)).

The neutralized base polymer is then dried with a belt, fluidized bed, tower dryer or drum dryer until the residual moisture content is preferably below 13% by weight, especially below 8% by weight and most preferably below 4% by weight, the water content being determined according to EDANA's recommended test method No. 430.2-02 "Moisture content" (EDANA = European Disposables and Nonwovens Association). The dried base polymer is thereafter ground and sieved, useful grinding apparatus typically include roll mills, pin mills, hammer mills, jet mills or swing mills.

The water-swellable polymers to be used can be post-crosslinked (surface crosslinked).

Useful post-crosslinkers include compounds comprising two or more groups capable of forming covalent bonds with the carboxylate groups of the polymers. The post-crosslinker is typically used in an amount of about 1.50 wt.% or less, preferably not more than 0.50% by weight, more preferably not more than 0.30% by weight and most preferably in the range from 0.001% and 0.15% by weight, all percentages being based on the base polymer, as an aqueous solution. It is possible to use a single post-crosslinker from the above selection or any desired mixtures of various post-crosslinkers.

The concentration of the at least one post-crosslinker v) in the aqueous post-crosslinking solution is for example in the range from 1% to 50% by weight, preferably in the range from 1.5% to 20% by weight and more preferably in the range from 2% to 5% by weight, based on the post-crosslinking solution.

It is, however, understood that post-crosslinkers which effect comparable surface-crosslinking results with respect to the final polymer performance may of course be used herein even when the water content of the solution containing such post-crosslinker and optionally a cosolvent is anywhere in the range of >0 to <100 % by weight.

The total amount of post-crosslinking solution based on the base polymer is typically in the range from 0.3% to 15% by weight and preferably in the range from 2% to 6% by weight. The practice of post-crosslinking is common knowledge to those skilled in the art and described for example in DE-A-12 239 074 and also prior German patent application 102004051242.6.

The water-swellable polymeric particles can have a particle size distribution in the range from 45 $\mu$m to 4000 $\mu$m. Particle sizes used in the hygiene sector preferably range from 45 $\mu$m to 1000 $\mu$m, preferably from 45 - 850 $\mu$m, and especially from 100 $\mu$m to 850 $\mu$m. It is preferable to use water-swellable polymeric particles having a narrow particle size distribution, especially 100 - 850 $\mu$m, or even 100 - 600$\mu$m

Narrow particle size distributions are those in which not less than 80% by weight of the particles, preferably not less than 90% by weight of the particles and most preferably not less than 95% by weight of the particles are within the selected range; this fraction can be determined using the familiar sieve method of EDANA 420.2-02 "Particle Size Distribution". Selectively, optical methods can be used as well, provided these are calibrated against the accepted sieve method of EDANA.

Preferred narrow particle size distributions have a span of not more than 700 $\mu$m, more preferably of not more than 600 $\mu$m, and most preferably of less than 400 $\mu$m. Span here refers to the difference between the coarse sieve and the fine sieve which bound the distribution. The coarse sieve is not coarser than 850 $\mu$m and the fine sieve is not finer than 45 $\mu$m. Particle size ranges which are preferred herein are, for example, fractions of 150 - 600 $\mu$m (span: 450 $\mu$m), of 200 - 700 $\mu$m (span: 500 $\mu$m), of 150 - 500 $\mu$m (span: 350 $\mu$m), of 150 - 300 $\mu$m (span: 150 $\mu$m), of 300 - 700 $\mu$m (span: 400 $\mu$m), of 400-800 $\mu$m (span: 400 $\mu$m), of 100 - 800 $\mu$m (span: 700 $\mu$m).

<u>Preferred processes tor making the water-swellable material</u>

For the water-swellable material herein that comprise core-shell particles as described herein, it is preferred that fluidized bed reactors are used to apply the shell, include for example the fluidized or suspended bed coaters familiar in the pharmaceutical industry. Particular preference is given to the Wurster process and the Glatt-Zeller process and these are described for example in "Pharmazeutische Technologie, Georg Thieme Verlag, 2nd edition (1989), pages 412-413" and also in "Arzneiformenlehre, Wissenschaftliche Verlagsbuchandlung mbH, Stuttgart 1985, pages 130-132". Particularly suitable batch and continuous fluidized bed processes on a commercial scale are described in Drying Technology, 20(2), 419-447 (2002).

In a preferred embodiment, a continuous fluidized bed process is used and the spray is operated in top or bottom-mode. In a particularly preferred embodiment the spray is operated bottom-mode and the process is continuous. A suitable apparatus is for example described in US 5,211,985. Suitable apparatus are available also for example from Glatt Maschinen- und Apparatebau AG (Switzerland) as series GF (continuous fluidized bed) and as ProCell® spouted bed. The spouted bed technology uses a simple slot instead of a screen bottom to generate the fluidized bed and is particularly suitable for materials which are difficult to fluidize.

Suitable fluidized bed reactors work according to the principle that the elastomeric polymer melt, solution or dispersion is finely atomized and the droplets randomly collide with the water-swellable polymer particles in a fluidized bed, whereby a substantially homogeneous shell builds up gradually and uniformly after many collisions. The size of the droplets must be inferior to the particle size of the water-swellable polymer. Droplet size is determined by the type of nozzle, the spraying conditions i.e., temperature, concentration, viscosity, pressure and typical droplets sizes are in the range 10 $\mu$m to 400 $\mu$m. A polymer particle size to droplet size ratio of at least 10 is typically observed. Small droplets with a narrow size distribution are favourable. The droplets of the atomized polymeric dispersion or solution are introduced either concurrently with the particle flow or from the side into the particle flow, and may also be sprayed from the top onto a fluidized bed. In this sense, other apparatus and equipment modifications which comply with this principle and which are likewise capable of building up fluidized beds are perfectly suitable for producing such effects.

The preferred process herein preferably utilizes Wurster Coaters. Examples for such coaters are PRECISION COATERS™ available from GEA-Aeromatic Fielder AG (Switzerland) and are accessible at Coating Place Inc. (Wisconsin, USA).

It is advantageous that the fluidized bed gas stream which enters is likewise chosen such that the total amount of the water-swellable polymeric particles is fluidized in the apparatus. The gas velocity for the fluidized bed is above the minimum fluidization velocity (measurement method described in Kunii and Levenspiel "Fluidization engineering" 1991) and below the terminal velocity of water-swellable polymer particles, preferably 10% above the minimum fluidization velocity. The gas velocity for the Wurster tube is above the terminal velocity of water-swellable polymer particles, usually below 100 m/s, preferably 10% above the terminal velocity.

The gas stream acts to vaporize the water, or the solvents. In a preferred embodiment, the coating conditions of gas stream and temperature are chosen so that the relative humidity or vapor saturation at the exit of the gas stream is in the range from 10% to 90%, preferably from 10% to 80%, or preferably from 10% to 70% and especially from 30% to 60%, based on the equivalent absolute humidity prevailing in the carrier gas at the same temperature or, if appropriate, the absolute saturation vapor pressure.

Coating may take place at a (product and/or carrier gas) temperature in the range from 0°C to 50°C, preferably at 5 - 45°C, especially 10 - 40°C and most preferably 15-35°C.

The temperature of the carrier gas leaving the coating step is typically not higher than 100°C, preferably lower than 60°C, more preferably lower than 50°C, even more preferably lower than 45°C, and most preferably lower than 40°C, but not lower than 0°C.

In a preferred embodiment, a deagglomerating aid is added before the heat-treating step (see below) to the particles to be coated or preferably which have already been coated. A deagglomerating aid would be known by those skilled in the art to be for example a finely divided water-insoluble salt selected from organic and inorganic salts and mixtures thereof, and also waxes and surfactants. A water-insoluble salt refers herein to a salt which at a pH of 7 has a solubility in water of less than 5 g/l, preferably less than 3 g/l, especially less than 2 g/l and most preferably less than 1 g/l (at 25°C and 1 bar). The use of a water-insoluble salt can reduce the tackiness due to the elastomeric polymer, especially the polyurethane which appears in the course of heat-treating.

The water-insoluble salts are used as a solid material or in the form of dispersions, preferably as an aqueous dispersion. It is particularly preferable to apply the deagglomerating aid after the elastomeric polymer has been applied and before the subsequent heat-treating step.

Suitable cations in the water-soluble salt are for example $Ca^{2+}$, $Mg^{2+}$, $Al^{3+}$, $Sc^{3+}$, $Y^{3+}$, $Ln^{3+}$ (where Ln denotes lanthanoids), $Ti^{4+}$, $Zr^{4+}$, $Li^+$, $K^+$, $Na^+$ or $Zn^{2+}$. Suitable inorganic anionic counterions are for example carbonate, sulfate, bicarbonate, orthophosphate, silicate, oxide or hydroxide. When a salt occurs in various crystal forms, all crystal forms of the salt shall be included. These deagglomerating aids can also be used in their hydrated forms. Useful deagglomerating aids further include many clays, talcum and zeolites. Silicon dioxide is preferably used in its amorphous form, for example as hydrophilic or hydrophobic Aerosil®, but selectively can also be used as aqueous commercially available silica sol, such as for example Levasil® Kiselsole (H.C. Starck GmbH), which have particle sizes in the range 5 - 75 nm.

The average particle size of the finely divided water-insoluble salt is typically less than 200 μm, preferably less than 100 μm, especially less than 50 μm, more preferably less than 20 μm, even more preferably less than 10 μm and most preferably in the range of less than 5 μm. Fumed silicas are often used as even finer particles, e.g. less than 50 nm, preferably less than 30 nm, even more preferably less than 20 nm primary particle size.

In a preferred embodiment, the finely divided water-insoluble salt is used in an amount in the range from 0.001% to 20% by weight, preferably less than 10% by weight, especially in the range from 0.001% to 5% by weight, more preferably in the range from 0.001% to 2% by weight and most preferably between 0.001 and 1% by weight, based on the weight of the water-swellable polymer.

In lieu of, or in addition to, the above inorganic salts it is also possible to use other known deagglomerating aids, examples being waxes and preferably micronized or preferably partially oxidized polyethylenic waxes, which can likewise be used in the form of an aqueous dispersion. Such waxes are described in EP 0 755 964, which is hereby expressly incorporated herein by reference.

Useful deagglomerating aids further include stearic acid, stearates - for example: magnesium stearate, calcium stearate, zinc stearate, aluminum stearate, and furthermore polyoxyethylene-20-sorbitan monolaurate and also polyethylene glycol 400 monostearate.

Useful deagglomerating aids likewise include surfactants. A surfactant can be used alone or mixed with one of the abovementioned deagglomerating aids, preferably a water-insoluble salt.

Heat-treating may take preferably place at temperatures above 50°C, preferably in a temperature range from 100 to 200°C, especially 120 - 160°C. In one embodiment, for the process steps of coating, heat treating, and cooling, it may be possible to use air or dried air in each of these steps.

In other embodiments, an inert gas may be used in one or more of these process steps.

In yet another embodiment, one can use mixtures of air and inert gas in one or more of these process steps.

The heat-treating is preferably carried out under inert gas. It is particularly preferable that the coating step be carried out under inert gas as well. It is very particularly preferable when the concluding cooling phase is carried out under protective gas too. Preference is therefore given to a process where the production of the water-swellable material may take place under inert gas.

Imperfections in the homogeneity of the coating or shell may be made by adding fillers in the coating solution or dispersion. Such imperfections may be useful in certain embodiments herein

After the heat-treating step has been concluded, the water-swellable material may be cooled. To this end, the warm and dry polymer is preferably continuously transferred into a downstream cooler.

Product temperature after cooling is typically less than 90°C, preferably less than 60°C, most preferably less than 40°C and preferably more than -20°C.

It may be preferable to use a fluidized bed cooler.

Preference is given to a water-swellable material obtainable by a process comprising the steps of :

a) spraying the water-swellable polymeric particles with a dispersion of an elastomeric polymer preferably at temperatures in the range from 0°C to 50°C;

b) optionally coating the particles obtained according to a), with a deagglomerating aid;

c) subsequently heat-treating the coated particles at a temperature above 50°C; and

d) subsequently cooling the heat-treated particles to below 90°C.

Useful solvents and dispersants for polyurethanes include solvents which make it possible to establish 1 to 40% by weight concentrations of the polyurethane in the respective solvent or mixture. As examples there may be mentioned alcohols, esters, ethers, ketones, amides, and halogenated hydrocarbons like methyl ethyl ketone, acetone, isopropanol, tetrahydrofuran, dimethylformamide, chloroform and mixtures thereof. Solvents which are polar, aprotic and boil below 100°C are particularly advantageous.

The polyurethane solution or dispersion applied by spray-coating is preferably very concentrated. For this, the viscosity of this polyurethane mixture must not be too high, or the polyurethane solution or dispersion can no longer be finely dispersed for spraying. Preference is given to a polyurethane solution or dispersion having a viscosity of <500 mPa·s, preferably of <300 mPa·s, more preferably of <100 mPa·s, even more preferably of <10 mPa·s, and most preferably < 5mPa·s (determined with a rotary viscometer at a shear rate ≥ 200 rpm for the polyurethane dispersion, Haake rotary viscometer type RV20, system M5, NV).

Aqueous herein refers to water and also mixtures of water with up to 20% by weight of water-miscible solvents, based on the total amount of solvent. Water-miscible solvents are miscible with water in the desired use amount at 25°C and 1 bar. They include alcohols such as methanol, ethanol, propanol, isopropanol, ethylene glycol, 1,2-propanediol, 1,3-propanediol, ethylene carbonate, glycerol and methoxyethanol.

Process Example 1 - Coating of ASAP 510 Z commercial product with Permax 120

The 800 - 850 μm fraction was sieved out of the commercially available product ASAP 510 Z (BASF AG) having the following properties and was then coated with Permax 120.

ASAP 510 Z (properties before sieving):

CRC = 29.0 g/g ; SFC = 50 $\times$10$^{-7}$ [cm$^3$s/g]

A Wurster laboratory coater was used, the amount of water-swellable polymer (ASAP 510 Z in this case) used was 500 g, the Wurster tube was 50 mm in diameter and 150 mm in length, the gap width (distance from base plate) was 15 mm, the Wurster apparatus was conical with a lower diameter of 150 mm expanding to an upper diameter of 300 mm, the carrier gas used was nitrogen having a temperature of 24°C, the gas speed was 3.1 m/s in the Wurster tube and 0.5 m/s in the surrounding annular space.

The elastomeric polymer dispersion was atomized using a nitrogen-driven two-material nozzle, opening diameter 1.2 mm, the nitrogen temperature being 28°C. The Permax 120 was sprayed from a 41% by weight neat aqueous dispersion whose temperature was 24°C, at a rate of 183 g of dispersion in the course of 65 min. In the process, 15% by weight of Permax was applied to the surface of the absorbent polymer. The amount reported is based on the water-swellable polymer used.

Two further runs were carried out in completely the same way except that the add-on level of the Permax was reduced: 5% by weight and 10% by weight.

The water-swellable material was subsequently removed and evenly distributed on Teflonized trays (to avoid sintering together) and dried in a vacuum cabinet at 150°C for 2 hours. Clumps were removed by means of a coarse sieve (1000 μm) and the polymers were characterized as follows:

| Loading with Permax 120 | CS-CRC [g/g] | CS-SFC [g/g] |
|---|---|---|
| 5% by weight | 27.4 | 764 |
| 10% by weight | 23.1 | 1994 |

(continued)

| Loading with Permax 120 | CS-CRC [g/g] | CS-SFC [g/g] |
|---|---|---|
| 15% by weight | 21.5 | 2027 |

Example 2 - Coating of ASAP 510 Z commercial product with Permax 200

1000 g ASAP 510 Z (BASF AG), as in example 1, is coated with Permax 200 using a Wurster laboratory coater as was used as in Example 1, but whereby the gas speed was 2.0 m/s in the Wurster tube and 0.5 m/s in the surrounding annular space, and the Permax 200 was sprayed from a 22% by weight neat aqueous dispersion whose temperature was 24°C, at a rate of 455 g of dispersion in the course of 168 min. In the process, 10% by weight of Permax was applied to the surface of the absorbent polymer. The amount reported is based on the water-swellable polymer used.

Three further runs were carried out in completely the same way except that the add-on level of the Permax was reduced: 2.5% by weight, 5.0% by weight and 7.5% by weight.

The water-swellable material was subsequently removed and evenly distributed on Teflonized trays (to avoid sintering together) and dried in a vacuum cabinet at 150°C for 2 hours. Clumps were removed by means of a coarse sieve (1000 $\mu$m) and the polymers were characterized as follows:

| Loading with Permax 200 | CS-CRC [g/g] | CS-SFC [g/g] |
|---|---|---|
| 2.5% by weight | 29.7 | 234 |
| 5.0% by weight | 27.5 | 755 |
| 7.5% by weight | 25.6 | 1082 |
| 10.0% by weight | 23.2 | 1451 |

Example 3 - Coating of ASAP 510 Z commercial product with Permax 200

1000 g ASAP 510 Z (BASF AG) with the commercially available particle size distribution of 150 - 850 $\mu$m was then coated with Permax 200, as in example 1, but with a the gas speed was 1.0 m/s in the Wurster tube and 0.26-0.30 m/s in the surrounding annular space and the nitrogen temperature being 25°C; the Permax 200 was sprayed from a 22% by weight neat aqueous dispersion whose temperature was 24°C, at a rate of 455 g of dispersion in the course of 221 min. In the process, 10% by weight of Permax was applied to the surface of the absorbent polymer. The amount reported is based on the water-swellable polymer used.

Three further runs were carried out in completely the same way except that the add-on level of the Permax was reduced: 2.5% by weight, 5.0% by weight and 7.5% by weight.

The water-swellable material was subsequently removed and evenly distributed on Teflonized trays (to avoid sintering together) and dried in a vacuum cabinet at 150°C for 2 hours. Clumps were removed by means of a coarse sieve (850 $\mu$m) and the polymers were characterized as follows:

| Loading with Permax 200 | CS-CRC [g/g] | CS-SFC [g/g] |
|---|---|---|
| 2.5% by weight | 25.5 | 279 |
| 5.0% by weight | 24.1 | 735 |
| 7.5% by weight | 23.1 | 930 |
| 10.0% by weight | 21.7 | 1303 |

Example 4: Use of a deagglomerating aid (calcium phosphate) before heat treatment

The run of Example 2 with 10% of Permax 200 was repeated. However, the polymer coated with the dispersion was transferred to a laboratory tumble mixer and 1.0% by weight of tricalcium phosphate type C13-09 (from Budenheim, Mainz) based on polymer was added and mixed dry with the coated polymer for about 10 minutes. Thereafter the polymer was transferred into a laboratory fluidized bed dryer (diameter about 70 mm) preheated to 150°C and, following a residence time of 30 minutes, the following properties were measured:

CS-CRC = 22.2 g/g; CS-SFC = 1483 $\times 10^{-7}$ [cm$^3$s/g]

There was no clumping whatsoever during the heat treatment in the fluidized bed, so that the fluidized bed remained very stable and as was demonstrated by subsequent sieving through a 1000 μm sieve.

Example 5: Use of a deagglomerating aid (Aerosil 90) before heat treatment

The run of Example 2 with 10% of Permax 200 was repeated. However, the water-swellable material was transferred to a laboratory tumble mixer and 1.0% by weight Aerosil 90 (from Degussa) based on water-swellable material was added and mixed dry with the water-swellable material for about 10 minutes. Thereafter the polymer was placed in a layer of 1.5 - 2.0 cm in an open glass 5 cm in diameter and 3 cm in height and heat treated in a forced-air drying cabinet at 150°C for 120 minutes. The material remained completely flowable, and did not undergo any caking or agglomeration.
The following properties were measured:

CS-CRC = 23.6 g/g; CS-SFC = 1677 $\times 10^{-7}$ [cm$^3$s/g]

The following is the procedure to make AM0127, as used in the examples below:
Unless stated, all compounds are obtained by Merck, and used w/o purification.
To 2000 g of glacial acrylic acid (AA), an appropriate amount of the core crosslinker (e.g., 1.284 g MethyleneBisAcrylAmide, MBAA, from Aldrich Chemicals) is added and allowed to dissolve at ambient temperature. An amount of water is calculated (6331 g) so that the total weight of all ingredients for the polymerization equals 10000 g (i.e., the concentration of AA is 20 w/w-%). 2000 mg of the initiator ("V50" = 2,2'-azobis (N,N'-dimethyleneisobutyramidine) dihydrochloride, from Waco Chemicals) are dissolved in approx. 40 ml of this calculated amount of the deionized water. 1665.3 g of 50% NaOH are weighted out separately in a Teflon or plastic beaker.
A 16,000 ml resin kettle (equipped with a four-necked glass cover closed with septa, suited for the introduction of a thermometer, syringe needles) is charged with ∼ 5 kg ice (prepared from de-ionized water - the amount of this ice is subtracted from the amount of DI water above) Typically, a magnetic stirrer, capable of mixing the whole content (when liquid), is added. The 50% NaOH is added to the ice, and the resulting slurry is stirred. Then, the acrylic acid/MBAA is added within 1 - 2 minutes, while stirring is continued, and the remaining water is added. The resulting solution is clear, all ice melted, and the resulting temperature is typically 15 - 25 °C. At this point, the initiator solution is added.
Then, the resin kettle is closed, and a pressure relief is provided, e.g., by puncturing two syringe needles through the septa. The solution is then spurged vigorously with argon via a 60 cm injection needle while stirring at ∼ 600 RPM. Stirring is discontinued after ∼ 10 minutes, while argon spurging is continued, and two photo lamps ("Twinlite") are placed on either side of the vessel. The solution typically starts to gel after 45 - 60 minutes total. At this point, persistent bubbles form on the surface of the gel, and the argon injection needle is raised above the surface of the gel. Purging with argon is continued at a reduced flow rate. The temperature is monitored; typically it rises from 20°C to 60 - 70°C within 60 - 90 minutes. Once the temperature drops below 60°C, the kettle is transferred into a circulation oven and kept at 60°C for 15 - 18 hours.
After this time, the resin kettle is allowed to cool, and the gel is removed into a flat glass dish. The gel is then broken or cut with scissors into small pieces, and transferred into a vacuum oven, where it is dried at 100°C/maximum vacuum. Once the gel has reached a constant weight (usually 3 days), it is ground using a mechanical mill (e.g., IKA mill), and sieved to 150 - 850 μm. At this point, parameters as used herein may be determined.

(This water-swellable polymer AM0127 had no post crosslinking.)

Further examples:

The following are other water-swellable materials made by the process described above in example 1, using the conditions and material specified in the table (ASAP 510 being available from BASF):

| Water-swellable material | Water-swellable polymer | Particle size (um) | Elastomeric polymer | Conc. Solvent | Coating Level by spraying | Max process temp (°C) | Coat time (min) |
|---|---|---|---|---|---|---|---|
| CP4-P120-15% | ASAP 510Z | 800-850 | Permax 120 | 41% water | 15% | 27.2 | 61.6 |

(continued)

| Water-swellable material | Water-swellable polymer | Particle size (um) | Elastomeric polymer | Conc. Solvent | Coating Level by spraying | Max process temp (°C) | Coat time (min) |
|---|---|---|---|---|---|---|---|
| CP9-P200-10% | ASAP 510Z | 800-850 | Permax 200 | 22% water | 10% | 29.4 | 81.9 |
| CP14-Xf-8.3% | ASAP 510Z | 800-850 | X-1007-040P | 5% THF | 8.30% | 32.8 | 99 |
| CP16-P200-10% | ASAP 510Z | 150-850 | Permax 200 | 22% water | 10% | 28.3 | 86 |
| CP27-P200-15%, 1% tricalcium phosphate | AM0127 | 600-850 | Permax 200 | 22% water | 15% | 30.6 | 105 |

The particle size distribution of the ASAP 510Z bulk material and the sieved fraction of ASAP510Z polymer particles with a particle size of 800-850 microns, 150-850 microns and 600-850 microns, as used above, is as follows:

| ASAP510Z (bulk distribution) | % | ASAP510z (800-850 um) | % |
|---|---|---|---|
| < 200 um | 7 % | 400 um | 4 % |
| 250- 300 um | 18 % | 500 um | 11 % |
| 350- 400 um | 33 % | 600 um | 25 % |
| 500 um | 20 % | 700 um | 33 % |
| 600 um | 12 % | 800 um | 25 % |
| 700 um | 5 % | TOTAL | 98 % |
| 800 um | 2 % | (mean: 700um) | |
| TOTAL | 97 % | | |

| ASAP510 150-850 | % | AM 0127 600-850 | % |
|---|---|---|---|
| 150um | 1.7% | <600 | 1.98% |
| 200um | 6.4% | 600um | 4.77% |
| 300um | 11.3% | 700um | 49.11% |
| 400um | 15.5% | 800um | 41.49% |
| 500um | 16.6% | 850um | 2.63% |
| 600um | 15.5% | | |
| 700um | 21.1% | | |
| 800um | 11.9% | | |

The materials obtained by the processes described above were submitted to the QUICS test, 4 hour CCRC test and CS-SFC test described herein and the values below were obtained. Also tested were some prior art materials, referred to as comparison water-swellable materials.

| Water-swellable material of the absorbent structures of the invention: | Annealing conditions | | SAC" | QUICS | CCRC | CS-SFC $10^{-7}$ cm$^3$ sec/g | ADI |
|---|---|---|---|---|---|---|---|
| CP4-P120-15%, ASAP510Z (800-850μm) | 2h 150°C | | 27.204 | 22.6 | 21.89 | 2324.2 | 5.88 |

(continued)

| Water-swellable material of the absorbent structures of the invention: | Annealing conditions | | SAC" | QUICS | CCRC | CS-SFC $10^{-7}$ cm$^3$ sec/g | ADI |
|---|---|---|---|---|---|---|---|
| CP9-P200-10%, ASAP510Z (8000-850μm) | 2h 150°C | | 30.569 | 24.2 | 23.79 | 1727.2 | 6.63 |
| CP14-Xf-8.3%, ASAP510Z (800-850μm) | 2h 150°C | | 29.122 | 32.0 | 21.60 | 1379.9 | 3.28 |
| CP16-P200-10%, ASAP510Z (150-850μm) | 2h 150°C | | 27.276 | 20.0 | 23.47 | 1356.5 | 4.85 |
| CP27-P200-15%, AM0127 (600-850μm) with the addition of 1%Tricalcium phosphate | 16h 150°C/2h 100°C | | 63.822 | 77.5 | 34.05 | 276.3 | 9.99 |
| Comparison water-swellable materials: | | | | | | | |
| W 52521# | 16h 150°C/2h 100°C | | 24.278 | 3.5 | 22.95 | 189.0 | 0.6 |
| AM 0127 base polymer 150-850um | 16h 150°C/2h 100°C | | 78.2 | -3.1 | | 0 | |
| 6% Vector 4211 on ASAP500 Base Polymer## | 2h 150°C | | 37.98 | 12.9 | | | |
| (1.6% VP654/6 on ### ASAP500 Base Polymer)### | 16h 150°C/2h 100°C | | 40.360 | 9.7 | | | |
| 6% Vector 4211 on ASAP500 Base Polymer## | 2h 150°C | | 35.018 | 10.6 | | | |
| (1.6% VP654/6 on ASAP500 Base Polymer)### | 16h 150°C/2h 100°C | | 37.58 | 8.1 | | | |
| # W52521: water-swellable material, containing water-swellable polymer particles, available from Stockhausen. ## water-swellable material as prepared in example 2.5 of co-pending application PCT application no.US2004/025836; "ASAP500 base polymer" available from BASF ### water-swellable material as prepared in example 2.5 of co-pending application PCT application no. US2004/025836; "ASAP500 base polymer" available from BASF | | | | | | | |

Test methods used herein:

4 hours Cylinder Centrifuge Retention Capacity (4 hours CCRC)

The Cylinder Centrifuge Retention Capacity (CCRC) method determines the fluid retention capacity of the water-swellable materials or polymers (sample) after centrifugation at an acceleration of 250g, herein referred to as absorbent capacity. Prior to centrifugation, the sample is allowed to swell in excess saline solution in a rigid sample cylinder with mesh bottom and an open top.

Duplicate sample specimens are evaluated for each material tested and the average value is reported.

The CCRC can be measured at ambient conditions, as set out in the QUICS test below, by placing the sample material (1.0 +/- 0.001 g) into a pre-weighed (+/-- 0.01 g) Plexiglas sample container that is open at the top and closed on the bottom with a stainless steel mesh (400) that readily allows for saline flow into the cylinder but contains the absorbent particles being evaluated. The sample cylinder approximates a rectangular prism with rounded-edges in the 67 mm height dimension. The base dimensions (78 X 58 mm OD, 67.2 X 47.2 MM ID) precisely match those of modular tube adapters, herein referred to as the cylinder stand, which fit into the rectangular rotor buckets (Heraeus # 75002252, VWR # 20300-084) of the centrifuge (Heraeus Megafuge 1.0; Heraeus # 75003491, VWR # 20300-016).

The loaded sample cylinders are gently shaken to evenly distribute the sample across the mesh surface and then placed upright in a pan containing saline solution. The cylinders should be positioned to ensure free flow of saline through the mesh bottom. Cylinders should not be placed against each other or against the wall of the pan, or sealed against the pan bottom. The sample is allowed to swell, without confining pressure and in excess saline, for 4 hours.

After 4 hours, the cylinders are immediately removed from the solution. Each cylinder is placed (mesh side down) onto a cylinder stand and the resulting assembly is loaded into the rotor basket such that the two sample assemblies are in balancing positions in the centrifuge rotor.

The samples are centrifuged for 3 minutes ($\pm$ 10s) after achieving the rotor velocity required to generate a centrifugal acceleration of 250 $\pm$ 5g at the bottom of the cylinder stand. The openings in the cylinder stands allow any solution expelled from the absorbent by the applied centrifugal forces to flow from the sample to the bottom of the rotor bucket where it is contained. The sample cylinders are promptly removed after the rotor comes to rest and weighed to the nearest 0.01 g.

The cylinder centrifuge retention capacity expressed as grams of saline solution absorbed per gram of sample material is calculated for each replicate as follows:

$$CCRC = \frac{m_{CS} - (m_{Cb} + m_S)}{m_S} \left[ \frac{g}{g} \right]$$

where:

$m_{CS}$:    is the mass of the cylinder with sample after centrifugation [g]
$m_{Cb}$:    is the mass of the dry cylinder without sample [g]
$m_S$:    is the mass of the sample without saline solution [g]

The CCRC referred to herein is the average of the duplicate samples reported to the nearest 0.01 g/g.

Quality Index for Core Shells (QUICS): method to calculate the QUICS value (QUICS method):

The water-swellable material herein is such that it allows effective absorption of fluids, whilst providing at the same time a very good permeability of the water-swellable material, once it has absorbed the fluids and once it is swollen, as for example may be expressed in CS-SFC value, described herein.

The inventors found that the change of the absorbent capacity of water-swellable material when it is submitted to grinding, is a measure to determine whether the water-swellable material exerts a pressure, which is high enough to ensure a much improved permeability of the water-swellable material (when swollen) of the absorbent structures of the invention, providing ultimately an improved performance in use.

Preferably, the water-swellable material comprises particles with a core-shell structure described herein, whereby the shell of elastomeric polymers exerts said significant pressure onto said core of water-swellable polymers (whilst still allowing high quantities of fluid to be absorbed). The inventors have found that without such a shell, the water-swellable material may have a good fluid absorbent capacity, but it will have a very poor permeability, in comparison to the water-swellable material of the absorbent structures of the invention. Thus, the inventors have found that this internal pressure that is generated by the shell is beneficial for the ultimate performance of water-swellable material herein.

Then, the change of the absorbent capacity of the water-swellable material, when the particles thereof are broken, e.g. when the shell on the particles (e.g., of the water-swellable polymers) is removed or destroyed, is a measure to determine whether the water-swellable material comprises particles with a shell that exerts a pressure onto the core, which is high enough to ensure a much improved permeability of the water-swellable material (when swollen) herein.

The following is the method used herein to determine the absorbent capacity of the water-swellable material, and the absorbent capacity of the same water-swellable material after submission to the grinding method (e.g. to destroy the shells), to subsequently determine the change of absorbent capacity, expressed as QUICS value.

As absorption fluid, a 0.9% NaCl solution in de-ionized water is used ('saline').

Each initial sample is 70 mg +/- 0.05 mg water-swellable material of the absorbent structures of the invention ('sample').

Duplicate sample specimens are evaluated for each material tested and the average value is used herein.

a. Determination of the Saline Absorbent Capacity (SAC) of the water-swellable material sample

At ambient temperature and humidity (i.e., 20°C and 50% +/- 10% humidity) and at ambient pressure, the sample is placed into a pre-weighed (+/-- 0.01 g) Plexiglas sample container (QUICS-pot) that is open at the top and closed on the bottom with a stainless steel mesh (400) that readily allows for saline flow into the cylinder but contains the absorbent particles being evaluated. The sample cylinder approximates a rectangular prism with rounded-edges in the 67 mm height dimension. The base dimensions (78 X 58 mm OD, 67.2 X 47.2 MM ID) precisely match those of modular tube adapters, herein referred to as the cylinder stand, which fit into the rectangular rotor buckets (Heraeus # 75002252, VWR # 20300-084) of the centrifuge (Heraeus Megafuge 1.0; Heraeus # 75003491, VWR # 20300-016).

The cylinder with sample is gently shaken to evenly distribute the sample across the mesh surface and it is then placed upright in a pan containing saline solution. A second cylinder with a second sample is prepared in the same manner. The cylinders should be positioned such that to free flow of saline through the mesh bottom is ensured at all times. The cylinders should not be placed against each other or against the wall of the pan, or sealed against the pan bottom. Each sample is allowed to swell, at the ambient conditions above, without confining pressure, for 4 hours. The saline level inside the cylinders is at least 3 cm from the bottom mesh. Optionally, a small amount of a dye may be added to stain the (elastic) shell, e.g., 10 PPM Toluidine Blue, or 10 PPM Chicago Sky Blue 6B.

After 4 hours (+/- 2 minutes), the cylinders are removed from the saline solution. Each cylinder is placed (mesh side down) onto a cylinder stand and the resulting assembly is loaded into the rotor basket of the centrifuge, such that the two sample assemblies are in balancing positions in the centrifuge rotor.

The samples are centrifuged for 3 minutes ($\pm$ 10s) after achieving the rotor velocity required to generate a centrifugal acceleration of 250 $\pm$ 5g at the bottom of the cylinder stand. The openings in the cylinder stands allow any solution expelled from the absorbent by the applied centrifugal forces to flow from the sample to the bottom of the rotor bucket where it is contained. The sample cylinders are promptly removed after the rotor comes to rest and weighed to the nearest 0.01 g.

The Saline Absorbent Capacity (SAC) expressed as grams of saline solution absorbed per gram of sample material is calculated for each replicate as follows:

$$SAC = \frac{m_{CS} - (m_{Cb} + m_S)}{m_S} \quad \left[\frac{g}{g}\right]$$

where:

$m_{CS}$:    is the mass of the cylinder with sample after centrifugation [g]
$m_{Cb}$:    is the mass of the dry cylinder without sample [g]
$m_{s}$:    is the mass of the sample without saline solution [g]

The SAC referred to herein is the average of the duplicate samples reported to the nearest 0.01 g/g.

b. Grinding of the sample:

After the weight measurements above, the swollen sample obtained above is transferred (under the same temperature, humidity and pressure conditions as set out above) to the centre of a flat Teflon sheet (20 * 20 cm * 1.0 mm) by means of a spatula. The Teflon sheet is supported on a hard, smooth surface, e.g., a standard laboratory bench. The QUICS-pot is weighed back to ensure that a > 95% transfer of the swollen sample to the Teflon sheet has been achieved.

A round glass plate (15 cm diameter, 8 mm thickness) is placed on top of the sample and the sample is thus squeezed between this top glass plate and the bottom support. Two 10 lb. weights are placed on the top glass plate; the top glass plate is rotated twice against the stationary Teflon sheet. (For example, when the water-swellable material comprises particles with shells, this operation will break or destroy the shell of the swollen particles of the swollen sample, and thus a (swollen) sample of broken particles, or typically particles with a broken or destroyed shell, are obtained.

c. Determination of the SAC" of the ground (swollen) sample obtained in 2. above:

The ground (swollen) sample obtained above in b) is quantitatively transferred back into the respective QUICS-pot, e.g. with the help of 0.9% NaCl solution from a squirt bottle, so that it is placed in the pot as described above. Each pot of each sample is placed in 0.9% NaCl solution under the same conditions and manner as above, but for 2 hours rather than 4 hours, and the second SAC" of the sample is determined by the centrifugation described above.

N.B.: The time elapsed between the end of the first centrifugation to determine the SAC (in step a.) and the beginning of the step c. to determine the SAC", (i.e., the start of transfer to QUICS pot), should not exceed more than 30 minutes.

d. QUICS calculation:

Then the QUICS as used herein is determined as follows:

$$QUICS = 100 * (SAC") / (SAC) - 100$$

CRC (Centrifuge Retention Capacity)

This method determines the free swellability of the water-swellable material or polymer in a teabag. To determine CRC, 0.2000 +/- 0.0050 g of dried polymer or material (particle size fraction 106 - 850 $\mu$m or as specifically indicated in the examples which follow) is weighed into a teabag 60 x 85 mm in size, which is subsequently sealed shut. The teabag is placed for 30 minutes in an excess of 0.9% by weight sodium chloride solution (at least 0.83 l of sodium chloride solution/1 g of polymer powder). The teabag is subsequently centrifuged at 250 g for 3 minutes. The amount of liquid is determined by weighing the centrifuged teabag. The procedure corresponds to that of EDANA recommended test method No. 441.2-02 (EDANA = European Disposables and Nonwovens Association). The teabag material and also the centrifuge and the evaluation are likewise defined therein.

CS-CRC (Core Shell Centrifuge Retention Capacity)

CS-CRC is carried out completely analogously to CRC, except that the sample's swelling time is extended from 30 min to 240 min.

Saline Flow Conductivity (SFC)

The method to determine the permeability of a swollen gel layer is the "Saline Flow Conductivity" also known as "Gel Layer Permeability" and is described in EP A 640 330. The equipment used for this method has been modified as described below.

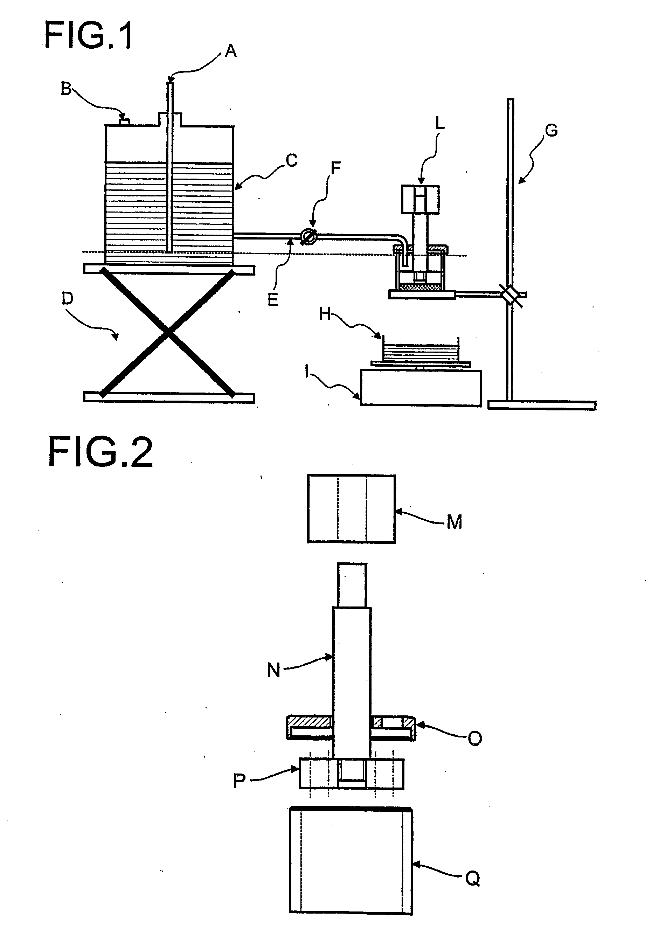

Figure 1 shows the permeability measurement equipment set-up with the open-ended tube for air admittance A, stoppered vent for refilling B, constant hydrostatic head reservoir C, Lab Jack D, delivery tube E, stopcock F, ring stand support G, receiving vessel H, balance I and the SFC apparatus L.

Figure 2 shows the SFC apparatus L consisting of the metal weight M, the plunger shaft N, the lid O, the center plunger P und the cylinder Q.

The cylinder Q has an inner diameter of 6.00 cm (area = 28.27 cm$^2$). The bottom of the cylinder Q is faced with a stainless-steel screen cloth (mesh width: 0.036 mm; wire diameter: 0.028 mm) that is bi-axially stretched to tautness prior to attachment. The plunger consists of a plunger shaft N of 21.15 mm diameter. The upper 26.0 mm having a diameter of 15.8 mm, forming a collar, a perforated center plunger P which is also screened with a stretched stainless-steel screen (mesh width: 0.036 mm; wire diameter: 0.028 mm), and annular stainless steel weights M. The annular stainless steel weights M have a center bore so they can slip on to plunger shaft and rest on the collar. The combined weight of the center plunger P, shaft and stainless-steel weights M must be 596 g ($\pm$ 6g), which corresponds to 0.30 PSI over the area of the cylinder. The cylinder lid O has an opening in the center for vertically aligning the plunger shaft N and a second opening near the edge for introducing fluid from the reservoir into the cylinder Q.

The cylinder Q specification details are:

Outer diameter of the Cylinder: 70.35 mm
Inner diameter of the Cylinder: 60.0 mm
Height of the Cylinder: 60.5 mm

The cylinder lid O specification details are:

Outer diameter of SFC Lid: 76.05 mm
Inner diameter of SFC Lid: 70.5 mm

Total outer height of SFC Lid: 12.7 mm
Height of SFC Lid without collar: 6.35 mm
Diameter of hole for Plunger shaft positioned in the center: 22.25 mm
Diameter of hole in SFC lid: 12.7 mm
Distance centers of above mentioned two holes: 23.5 mm

The metal weight M specification details are:

Diameter of Plunger shaft for metal weight: 16.0 mm
Diameter of metal weight: 50.0 mm
Height of metal weight: 39.0 cm

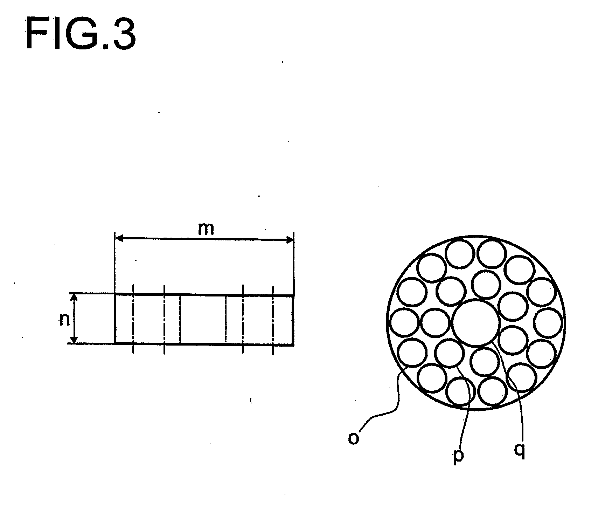

Figure 3 shows the plunger center P specification details:

Diameter m of SFC Plunger center: 59.7 mm
Height n of SFC Plunger center: 16.5 mm
14 holes o with 9.65 mm diameter equally spaced on a 47.8 mm bolt circle and 7 holes p with a diameter of 9.65 mm equally spaced on a 26.7 mm bolt circle 5/8 inches thread q

Prior to use, the stainless steel screens of SFC apparatus, should be accurately inspected for clogging, holes or over stretching and replaced when necessary. An SFC apparatus with damaged screen can deliver erroneous SFC results, and must not be used until the screen has been fully replaced.
Measure and clearly mark, with a permanent fine marker, the cylinder at a height of 5.00 cm ($\pm$ 0.05 cm) above the screen attached to the bottom of the cylinder. This marks the fluid level to be maintained during the analysis. Maintenance of correct and constant fluid level (hydrostatic pressure) is critical for measurement accuracy.
A constant hydrostatic head reservoir C is used to deliver NaCl solution to the cylinder and maintain the level of solution at a height of 5.0 cm above the screen attached to the bottom of the cylinder. The bottom end of the reservoir air-intake tube A is positioned so as to maintain the fluid level in the cylinder at the required 5.0 cm height during the measurement, i.e., the height of the bottom of the air tube A from the bench top is the same as the height from the bench top of the 5.0 cm mark on the cylinder as it sits on the support screen above the receiving vessel. Proper height alignment of the air intake tube A and the 5.0 cm fluid height mark on the cylinder is critical to the analysis. A suitable reservoir consists of a jar containing: a horizontally oriented L-shaped delivery tube E for fluid delivering, an open-ended vertical tube A for admitting air at a fixed height within the reservoir, and a stoppered vent B for re-filling the reservoir. The delivery tube E, positioned near the bottom of the reservoir C, contains a stopcock F for starting/stopping the delivery of fluid. The outlet of the tube is dimensioned to be inserted through the opening in the cylinder lid O, with its end positioned below the surface of the fluid in the cylinder (after the 5 cm height is attained). The air-intake tube is held in place with an o-ring collar. The reservoir can be positioned on a laboratory jack D in order to adjust its height relative to that of the cylinder. The components of the reservoir are sized so as to rapidly fill the cylinder to the required height (i.e., hydrostatic head) and maintain this height for the duration of the measurement. The reservoir must be capable to deliver liquid at a flow rate of minimum 3 g/sec for at least 10 minutes.
Position the plunger/cylinder apparatus on a ring stand with a 16 mesh rigid stainless steel support screen (or equivalent). This support screen is sufficiently permeable so as to not impede fluid flow and rigid enough to support the stainless steel mesh cloth preventing stretching. The support screen should be flat and level to avoid tilting the cylinder apparatus during the test. Collect the fluid passing through the screen in a collection reservoir, positioned below (but not supporting) the support screen. The collection reservoir is positioned on a balance accurate to at least 0.01 g. The digital output of the balance is connected to a computerized data acquisition system. ,

Preparation of reagents

Following preparations are referred to a standard 1 liter volume. For preparation multiple than 1 liter, all the ingredients must be calculated as appropriate.

Jayco Synthetic Urine

Fill a 1L volumetric flask with de-ionized water to 80% of its volume, add a stir bar and put it on a stirring plate. Separately, using a weighing paper or beaker weigh (accurate to $\pm$ 0.01 g) the amounts of the following dry ingredients using the analytical balance and add them into the volumetric flask in the same order as listed below. Mix until all the solids are dissolved then remove the stir bar and dilute to 1L volume with distilled water. Add a stir bar again and mix on a stirring plate for a few minutes more. The conductivity of the prepared solution must be 7.6 ± 0.23 mS/cm.

Chemical Formula Anhydrous Hydrated

Potassium Chloride (KCl) 2.00 g
Sodium Sulfate (Na2SO4) 2.00 g
Ammonium dihydrogen phosphate (NH4H2PO4) 0.85 g
Ammonium phosphate, dibasic ((NH4)2HPO4) 0.15 g
Calcium Chloride (CaCl2) 0.19 g (2 H2O) 0.25 g
Magnesium chloride (MgCl2) 0.23 g (6 H2O) 0.50 g

To make the preparation faster, wait until total dissolution of each salt before adding the next one. Jayco may be stored in a clean glass container for 2 weeks. Do not use if solution becomes cloudy. Shelf life in a clean plastic container is 10 days.

<u>0.118 M Sodium Chloride (NaCl) Solution</u>

Using a weighing paper or beaker weigh (accurate to ± 0.01 g) 6.90 g of sodium chloride into a 1L volumetric flask and fill to volume with de-ionized water. Add a stir bar and mix on a stirring plate until all the solids are dissolved. The conductivity of the prepared solution must be 12.50 ± 0.38 mS/cm.

<u>Test preparation</u>

Using a reference metal cylinder (40 mm diameter; 140 mm height) set the caliper gauge (e.g., Mitotoyo Digimatic Height Gage) to read zero. This operation is conveniently performed on a smooth and level bench top. Position the SFC apparatus without water-swellable material or water-swellable polymer ('sample') under the caliper gauge and record the caliper as L1 to the nearest of 0.01 mm.
Fill the constant hydrostatic head reservoir with the 0.118 M NaCl solution. Position the bottom of the reservoir air-intake tube A so as to maintain the top part of the liquid meniscus in the SFC cylinder at the required 5.0 cm height during the measurement. Proper height alignment of the air-intake tube A at the 5 cm fluid height mark on the cylinder is critical to the analysis.
Saturate an 8 cm fritted disc (7 mm thick; e.g., Chemglass Inc. # CG 201- 51, coarse porosity) by adding excess synthetic urine on the top of the disc. Repeating until the disc is saturated. Place the saturated fritted disc in the hydrating dish and add the synthetic urine until it reaches the level of the disc. The fluid height must not exceed the height of the disc.
Place the collection reservoir on the balance and connect the digital output of the balance to a computerized data acquisition system. Position the ring stand with a 16 mesh rigid stainless steel support screen above the collection dish. This 16 mesh screen should be sufficiently rigid to support the SFC apparatus during the measurement. The support screen must be flat and level.

<u>Sampling</u>

Samples should be stored in a closed bottle and kept in a constant, low humidity environment. Mix the sample to evenly distribute particle sizes. Remove a representative sample to be tested from the center of the container using the spatula. The use of a sample divider is recommended to increase the homogeneity of the sample particle size distribution.

<u>SFC procedure</u>

Position the weighing funnel on the analytical balance plate and zero the balance. Using a spatula weigh 0.9 g (± 0.05g) of the sample into the weighing funnel. Position the SFC cylinder on the bench, take the weighing funnel and gently, tapping with finger, transfer the sample into the cylinder being sure to have an evenly dispersion of it on the screen. During the sample transfer, gradually rotate the cylinder to facilitate the dispersion and get homogeneous distribution. It is important to have an even distribution of particles on the screen to obtain the highest precision result. At the end of the distribution the sample material must not adhere to the cylinder walls. Insert the plunger shaft into the lid central hole then insert the plunger center into the cylinder for few centimeters. Keeping the plunger center away from sample, insert the lid in the cylinder and carefully rotate it until the alignment between the two is reached. Carefully rotate the plunger to reach the alignment with lid then move it down allowing it to rest on top of the dry sample. Insert the stainless steel weight to the plunger rod and check if the lid moves freely. Proper seating of the lid prevents binding and assures an even distribution of the weight on the gel bed.

The thin screen on the cylinder bottom is easily stretched. To prevent stretching, apply a sideways pressure on the plunger rod, just above the lid, with the index finger while grasping the cylinder portion of the apparatus. This "locks" the plunger in place against the inside of the cylinder so that the apparatus can be lifted. Place the entire apparatus on the fritted disc in the hydrating dish. The fluid level in the dish should not exceed the height of the fritted disc. Care should be taken so that the layer does not loose fluid or take in air during this procedure. The fluid available in the dish should be enough for all the swelling phase. If needed, add more fluid to the dish during the hydration period to ensure there is sufficient synthetic urine available. After a period of 60 minutes, place the SFC apparatus under the caliper gauge and record the caliper as L2 to the nearest of 0.01 mm. Calculate, by difference L2 - L1, the thickness of the gel layer as L0 to the nearest $\pm$ 0.1 mm. If the reading changes with time, record only the initial value.

Transfer the SFC apparatus to the support screen above the collection dish. Be sure, when lifting the apparatus, to lock the plunger in place against the inside of the cylinder. Position the constant hydrostatic head reservoir such that the delivery tube is placed through the hole in the cylinder lid. Initiate the measurement in the following sequence:

> a) Open the stopcock of the constant hydrostatic head reservoir and permit the fluid to reach the 5 cm mark. This fluid level should be obtained within 10 seconds of opening the stopcock.
> b) Once 5 cm of fluid is attained, immediately initiate the data collection program.

With the aid of a computer attached to the balance, record the quantity of fluid passing through the gel layer versus time at intervals of 20 seconds for a time period of 10 minutes. At the end of 10 minutes, close the stopcock on the reservoir. The data from 60 seconds to the end of the experiment are used in the calculation. The data collected prior to 60 seconds are not included in the calculation. Perform the test in triplicate for each sample.

Evaluation of the measurement remains unchanged from EP-A 640 330. Through-flux is captured automatically.

Saline flow conductivity (SFC) is calculated as follows:

$$\text{SFC [cm}^3\text{s/g]} = (Fg(t{=}0) \times L_0) / (d \times A \times WP),$$

where Fg(t=0) is the through-flux of NaCl solution in g/s, which is obtained from a linear regression analysis of the Fg(t) data of the through-flux determinations by extrapolation to t=0, $L_0$ is the thickness of the gel layer in cm, d is the density of the NaCl solution in $g/cm^3$, A is the area of the gel layer in $cm^2$ and WP is the hydrostatic pressure above the gel layer in $dyn/cm^2$.

### CS-SFC (Core Shell Saline Flow Conductivity)

CS-SFC is determined completely analogously to SFC, with the following changes:

To modify the SFC the person skilled in the art will design the feed line including the stopcock in such a way that the hydrodynamic resistance of the feed line is so low that prior to the start of the measurement time actually used for the evaluation an identical hydrodynamic pressure as in the SFC (5 cm) is attained and is also kept constant over the duration of the measurement time used for the evaluation.

- the weight of sample used is 1.50 +/- 0.05 g
- a 0.9% by weight sodium chloride solution is used as solution to preswell the sample and for through-flux measurement
- the preswell time of the sample for measurement is 240 minutes
- for preswelling, a filter paper 90 mm in diameter (Schleicher & Schüll, No 597) is placed in a 500 ml crystallizing dish (Schott, diameter = 115 mm, height = 65 mm) and 250 ml of 0.9% by weight sodium chloride solution are added, then the SFC measuring cell with the sample is placed on the filter paper and swelling is allowed for 240 minutes
- the through-flux data are recorded every 5 seconds, for a total of 3 minutes
- the points measured between 10 seconds and 180 seconds are used for evaluation and Fg(t=0) is the through-flux of NaCl solution in g/s which is obtained from a linear regression analysis of the Fg(t) data of the through-flux determinations by extrapolation to t=0
- the stock reservoir bottle in the SFC-measuring apparatus for through-flux solution contains about 5L of sodium chloride solution.

Preparation of films of the elastic polymer

In order to subject the elastic polymer used herein to some of the test methods below, films need to be obtained of said polymers.

The preferred average (as set out below) caliper of the (dry) films for evaluation in the test methods herein is around 60 μm.

Methods to prepare films are generally known to those skilled in the art and typically comprise solvent casting, hotmelt extrusion or melt blowing films. Films prepared by these methods may have a machine direction that is defined as the direction in which the film is drawn or pulled. The direction perpendicular to the machine direction is defined as the cross-direction.

For the purpose of the invention, the films used in the test methods below are formed by solvent casting, except when the elastic polymer cannot be made into a solution or dispersion of any of the solvents listed below, and then the films are made by hotmelt extrusion as described below. (The latter is the case when particulate matter from the elastic film-forming polymer is still visible in the mixture of the material or coating agent and the solvent, after attempting to dissolve or disperse it at room temperature for a period between 2 to 48 hours, or when the viscosity of the solution or dispersion is too high to allow film casting.)

The resulting film should have a smooth surface and be free of visible defects such as air bubbles or cracks.

An example to prepare a solvent cast film herein from an elastomeric polymer:

The film to be subjected to the tests herein can be prepared by casting a film from a solution or dispersion of said polymer as follows:

The solution or dispersion is prepared by dissolving or dispersing the elastomeric polymer, at 10 weight%, in water, or if this is not possible, in THF (tetrahydrofuran), or if this is not possible, in dimethylformamide (DMF), or if this is not possible in methyl ethyl ketone (MEK), or if this is not possible, in dichloromethane or if this is not possible in toluene, or if this is not possible in cyclohexane (and if this is not possible, the hotmelt extrusion process below is used to form a film). Next, the dispersion or solution is poured into a Teflon dish and is covered with aluminum foil to slow evaporation, and the solvent or dispersant is slowly evaporated at a temperature above the minimum film forming temperature of the polymer, typically about 25°C, for a long period of time, e.g., during at least 48 hours, or even up to 7 days. Then, the films are placed in a vacuum oven for 6 hours, at 25°C, to ensure any remaining solvent is removed.

The process to form a film from an aqueous dispersion is as follows:

The dispersion may be used as received from the supplier, or diluted with water as long as the viscosity remains high enough to draw a film (200 - 500 cps). The dispersion solution (5 - 10 mL) is placed onto a piece of aluminum foil that is attached to the stage of the draw down table. The polymer dispersion is drawn using a Gardner metering rod #30 or #60 to draw a film that is 50-100 microns thick after drying. The dispersant is slowly evaporated at a temperature above the minimum film forming temperature of the polymer, typically about 25°C, for a long period of time, e.g., during at least 48 hours, or even up to 7 days. The film is heated in a vacuum oven at 150 °C for a minimum of 5 minutes up to 2h, then the film is removed from the foil substrate by soaking in warm water bath for 5 to 10 min to remove the films from the substrate. The removed film is then placed onto a Teflon sheet and dried under ambient conditions for 24h. The dried films are then sealed in a plastic bag until testing can be performed.

The process to prepare a hotmelt extruded film herein is as follows:

If the solvent casting method is not possible, films of the elastomeric polymer 1 herein may be extruded from a hot melt using a rotating single screw extrusion set of equipment operating at temperatures sufficiently high to allow the elastic film-forming polymer to flow. If the polymer has a melting temperature Tm, then the extrusion should take place at least 20 K above said Tm. If the polymer is amorphous (i.e., does not have a Tm), steady shear viscometry can be performed to determine the order to disorder transition for the polymer, or the temperature where the viscosity drops dramatically. The direction that the film is drawn from the extruder is defined as the machine direction and the direction perpendicular to the drawing direction is defined as the cross direction.

Heat-treating of the films:

The heat-treating of the films should, for the purpose of the test methods below, be done by placing the film in a vacuum oven at a temperature which is about 20 K above the highest Tg of the used elastomeric polymer, and this is done for 2 hours in a vacuum oven at less than 0.1 Torr, provided that when the elastic film-forming polymer has a melting temperature Tm, the heat-treating temperature is at least 20 K below the Tm, and then preferably (as close to) 20 K above the highest Tg. When the Tg is reached, the temperature should be increased slowly above the highest Tg to avoid gaseous discharge that may lead to bubbles in the film. For example, a material with a hard segment Tg of 70 °C might be heat-treated at 90 °C for 10 min, followed by incremental increases in temperature until the heat-treating temperature is reached.

If the elastomeric polymer has a Tm, then said heat-treating of the films (prepared as set out above and to be tested by the methods below) is done at a temperature which is above the (highest) Tg and at least 20 K below the Tm and (as close to) 20 K above the (highest) Tg. For example, a wet-extensible material that has a Tm of 135°C and a highest Tg (of the hard segment) of 100°C, would be heat-treated at 115°C.

In the absence of a measurable Tg or Tm, the temperature for heat-treating in this method is the same as used in the process for making water-absorbing material.

Removal of films, if applicable

If the dried and optionally heat-treated films are difficult to remove from the film-forming substrate, then they may be placed in a warm water bath for 30 s to 5 min to remove the films from the substrate. The film is then subsequently dried for 6 - 24h at 25°C.

Moisture Vapor Transmission Rate Method (MVTR method)

MVTR method measures the amount of water vapor that is transmitted through a film (e.g. of the shell material or elastomeric polymers described herein) under specific temperature and humidity, e.g., ambient as described herein. The transmitted vapor is absorbed by $CaCl_2$ desiccant and determined gravimetrically. Samples are evaluated in triplicate, along with a reference film sample of established permeability (e.g., Exxon Exxaire microporous material #XBF-110W) that is used as a positive control.

This test uses a flanged cup (machined from Delrin (McMaster-Carr Catalog #8572K34) and anhydrous $CaCl_2$ (Wako Pure Chemical Industries, Richmond, Va.; Catalog 030-00525). The height of the cup is 55 mm with an inner diameter of 30 mm and an outer diameter of 45 mm. The cup is fitted with a silicone gasket and lid containing 3 holes for thumb screws to completely seal the cup. Desiccant particles are of a size to pass through a No. 8 sieve but not through a No. 10 sieve. Film specimens approximately 1.5" x 2.5" that are free of obvious defects are used for the analysis. The film must completely cover the cup opening, A, which is 0.0007065 $m^2$.

The cup is filled with anhydrous $CaCl_2$ to within 1 cm of the top. The cup is tapped on the counter 10 times, and the $CaCl_2$ surface is leveled. The amount of $CaCl_2$ is adjusted until the headspace between the film surface and the top of the CaCl2 is 1.0 cm. The film is placed on top of the cup across the opening (30 mm) and is secured using the silicone gasket, retaining ring, and thumb screws. Properly installed, the specimen should not be wrinkled or stretched. The sample assembly is weighed with an analytical balance and recorded to $\pm$ 0.001 g. The assembly is placed in a constant temperature (40 $\pm$ 3°C) and humidity (75 $\pm$ 3% RH) chamber for 5.0 hr $\pm$ 5 min. The sample assembly is removed, covered with Saran Wrap® and is secured with a rubber band. The sample is equilibrated to room temperature for 30 min, the plastic wrap removed, and the assembly is reweighed and the weight is recorded to $\pm$ 0.001 g. The absorbed moisture $M_a$ is the difference in initial and final assembly weights. MVTR, in $g/m^2/24hr$ ($g/m^2/day$), is calculated as:

$$\text{MVTR} = M_a / (A * 0.208 \text{ day})$$

Replicate results are averaged and rounded to the nearest 100 $g/m^2/24hr$, e.g., 2865 $g/m^2/24hr$ is herein given as 2900 $g/m^2/24hr$ and 275 $g/m^2/24hr$ is given as 300 $g/m^2/24hr$.

Glass Transition Temperatures

Glass Transition Temperatures (Tg's) are determined for the purpose of this invention by differential scanning calorimetry (DSC). The calorimeter should be capable of heating/cooling rates of at least 20°C/min over a temperature range, which includes the expected Tg's of the sample that is to be tested, e.g., of from -90° to 250°C, and the calorimeter should have a sensitivity of about 0.2 $\mu$W. TA Instruments Q1000 DSC is well-suited to determining the Tg's referred to herein. The material of interest can be analyzed using a temperature program such as: equilibrate at -90°C, ramp at 20°C/min to 120°C, hold isothermal for 5 minutes, ramp 20°C/min to -90°C, hold isothermal for 5 minutes, ramp 20°C/min to 250°C. The data (heat flow versus temperature) from the second heat cycle is used to calculate the Tg via a standard half extrapolated heat capacity temperature algorithm. Typically, 3-5 g of a sample material is weighed (+/- 0.1 g) into an aluminum DSC pan with crimped lid.

Elastomeric Polymer Molecular Weights

Gel Permeation Chromatography with Multi-Angle Light Scattering Detection (GPC-MALS) may be used for determining the molecular weight of the elastomeric polymers (e.g., of the shells herein). Molecular weights referred to herein are the weight-average molar mass (Mw). A suitable system for making these measurements consists of a DAWN DSP Laser Photometer (Wyatt Technology), an Optilab DSP Interferometric Refractometer (Wyatt Technology), and a standard HPLC pump, such as a Waters 600E system, all run via ASTRA software (Wyatt Technology).

As with any chromatographic separation, the choice of solvent, column, temperature and elution profiles and conditions depends upon the specific polymer which is to be tested. The following conditions have been found to be generally applicable for the elastomeric polymers referred to herein: Tetrahydrofuran (THF) is used as solvent and mobile phase; a flow rate of 1 mL/min is passed through two 300 x 7.5mm, 5$\mu$m, PLgel, Mixed-C GPC columns (Polymer Labs) which are placed in series and are heated to 40-45°C (the Optilab refractometer is held at the same temperature); 100 $\mu$L of a 0.2% polymer solution in THF solution is injected for analysis. The dn/dc values are obtained from the literature where available or calculated with ASTRA utility. The weight-average molar mass (Mw) is calculated by with the ASTRA software using the Zimm fit method.

Pulsed NMR method to determine weight percentage of the shell

The following describes the method, which can be used to determine the weight percentage of the shell (by weight of the sample of the water-swellable material) of the water-swellable particles of said material, whereby said shell comprises elastomeric polymers with (at least one) Tg of less than 60°C, using known Pulsed Nuclear Magnetic Resonance techniques, whereby the size of each spin-echo signal from identical protons (bonded to the molecules of said elastomeric polymer present in a sample) is a measure of the amount of said protons present in the sample and hence a measure of the amount of said molecules of said elastomeric polymer present (and thus the weight percentage thereof- see below) present in the sample.

For the pulsed NMR measurement a Maran 23 Pulsed NMR Analyzer with 26 mm Probe, Universal Systems, Solon, OH, may be used.

The sample will be a water-swellable material, of which its chemical composition is know, and of which the weight percentage of the shell is to be determined.

To generate a calibration curve for needed for this measurement, water-swellable materials of the same chemical composition, but with known shell weight percentage levels are prepared as follows: 0% (no shell), 1%, 2%, 3%, 4%, 6%, 8% and 10% by weight. These are herein referred to as 'standards'.

Each standard and the sample must be vacuum dried for 24h at 120°.C before the start of a measurement.

For each measurement, 5 grams (with an accuracy of 0.0001g) of a standard or of a sample is weighed in a NMR tube (for example Glass sample tubes, 26 mm diameter, at least 15 cm in height).

The sample and the eight standards are placed in a mineral oil dry bath for 45 minutes prior to testing, said dry bath being set at 60°C +/- 1°C. (The bath temperature is verified by placing a glass tube containing two inches of mineral oil and a thermometer into the dry bath.) For example, a Fisher Isotemp. Dry Bath Model 145, 120V, 50/60 HZ, Cat. #11-715-100, or equivalent can be used.

The standards and the sample should not remain in the dry bath for more than 1 hour prior to testing. The sample and the standards must be analyzed within 1 minute after transfer from the bath to the NMR instrument.

For the NMR measurements, the NMR and RI Multiquant programs of the NMR equipment are started and the measurements are made following normal procedures (and using the exact shell amount [g] for each standard in the computer calculations). The centre of the spin echo data is used when analyzing the data, using normal procedures.

Then, the sample, prepared as above, is analyzed in the same manner and using the computer generated data regarding the standards, the weight percentage of the shell of the sample can be calculated.

Wet-tensile-stress Test:

This test method is used to measure the wet-elongation at break (= extensibility at break) and tensile properties of films of elastomeric polymers as used herein, by applying a uniaxial strain to a flat sample and measuring the force that is required to elongate the sample. The film samples are herein strained in the cross-direction, when applicable.

A preferred piece of equipment to do the tests is a tensile tester such as a MTS Synergie100 or a MTS Alliance available from MTS Systems Corporation 14000 Technology Drive, Eden Prairie, MN, USA, with a 25N or 50N load cell. This measures the Constant Rate of Extension in which the pulling grip moves at a uniform rate and the force measuring mechanisms moves a negligible distance (less than 0.13mm) with increasing force. The load cell is selected such that the measured loads (e.g., force) of the tested samples will be between 10 and 90% of the capacity of the load cell.

Each sample is die-cut from a film, each sample being 1 x 1 inch (2.5 x 2.5 cm), as defined above, using an anvil hydraulic press die to cut the film into sample(s) (Thus, when the film is made by a process that does not introduce any orientation, the film may be tested in either direction.). Test specimens (minimum of three) are chosen which are substantially free of visible defects such as air bubbles, holes, inclusions, and cuts. They must also have sharp and substantially defect-free edges.

The thickness of each dry specimen is measured to an accuracy of 0.001 mm with a low pressure caliper gauge such as a Mitutoyo Caliper Gauge using a pressure of about 0.1 psi. Three different areas of the sample are measured and the average caliper is determined. The dry weight of each specimen is measured using a standard analytical balance to an accuracy of 0.001 g and recorded. Dry specimens are tested without further preparation for the determination of dry-elongation, dry-secant modulus, and dry-tensile stress values used herein.

For wet testing, pre-weighed dry film specimens are immersed in saline solution [0.9% (w/w) NaCl] for a period of 24 hours at ambient temperature (23 +/- 2°C). Films are secured in the bath with a 120-mesh corrosion-resistant metal screen that prevents the sample from rolling up and sticking to itself. The film is removed from the bath and blotted dry with an absorbent tissue such as a Bounty® towel, to remove excess or non-absorbed solution from the surface. The wet caliper is determined as noted for the dry samples. Wet specimens are used for tensile testing without further preparation. Testing should be completed within 5 minutes after preparation is completed. Wet specimens are evaluated to determine wet-elongation, wet-secant modulus, and wet-tensile stress.

Tensile testing is performed on a constant rate of extension tensile tester with computer interface such as an MTS Alliance tensile tester with Testworks 4 software. Load cells are selected such that measured forces fall within 10-90% of the cell capacity. Pneumatic jaws, fitted with flat 1"-square rubber-faced grips, are set to give a gage length of 1 inch. The specimen is loaded with sufficient tension to eliminate observable slack, but less than 0.05N. The specimens are extended at a constant crosshead speed of 10"/min until the specimen completely breaks. If the specimen breaks at the grip interface or slippage within the grips is detected, then the data is disregarded and the test is repeated with a new specimen and the grip pressure is appropriately adjusted. Samples are run in triplicate to account for film variability.

The resulting tensile force-displacement data are converted to stress-strain curves using the initial sample dimensions from which the elongation, tensile stress, and modulus that are used herein are derived. The average secant modulus at 400% elongation is defined as the slope of the line that intersects the stress-strain curve at 0% and 400% strain. Three stress-strain curves are generated for each extensible film coating that is evaluated. The modulus used herein is the average of the respective values derived from each curve.

<u>Determination of the shell caliper and shell caliper uniformity</u>

The elastomeric shells on water-swellable polymers or particles thereof, as used herein, can typically be investigated by standard scanning electron microscopy, preferably environmental scanning electron microscopy (ESEM) as known to those skilled in the art. In the following method the ESEM evaluation is also used to determine the average shell caliper and the shell caliper uniformity, of the shells of the particles of the water-swellable materials herein, via cross-section of the particles.

Equipment model: ESEM XL 30 FEG (Field Emission Gun)
ESEM setting : high vacuum mode with gold covered samples to obtain also images at low magnification (35X) and ESEM dry mode with LFD (large Field Detector which detects ~80% Gasous Secondary Electrons + 20% Secondary Electrons) and bullet without PLA (Pressure Limiting Aperture) to obtain images of the shells as they are (no gold coverage required).
Filament Tension: 3KV in high vacuum mode and 12 KV in ESEM dry mode.
Pressure in Chamber on the ESEM dry mode: from 0.3 Torr to 1 Torr on gelatinous samples and from 0.8 to 1 Torr for other samples.

Each sample can be observed after about 1 hour at 20°C, 80% relative humidity using the standard ESEM conditions/ equipment. Also, a sample of a particle without shell can thus be observed, as reference. Then, the same samples can be observed in high vacuum mode. Then each sample can be cut via a cross-sectional cut with a Teflon blade (Teflon blades are available from the AGAR scientific catalogue (ASSING) with reference code T5332), and observed again under vacuum mode.

The shells are clearly visible in the ESEM images, in particular when observing the cross-sectional views.

The average shell caliper is determined by analyzing at least 5 particles of the water-swellable material, comprising said shell, and determining 5 average calipers, one average per particle (and each of those averages is obtained by analyzing the cross-section of each particle and measuring the caliper of the shell in at least 3 different areas) and taking then the average of these 5 average calipers.

The uniformity of the shell is determined by determining the minimum and maximum caliper of the shell via ESEM of the cross-sectional cuts of at least 5 different particles and determining the average (over 5) minimum and average maximum caliper and the ratio thereof.

If the shell is not clearly visible in ESEM, then staining techniques known to the skilled in the art that are specific for the shell applied may be used such as enhancing the contrast with osmium tetraoxide, potassium permanganate and the like, e.g., prior to using the ESEM method.

Theoretical Equivalent Shell Caliper of the particles of the water-swellable material herein

If the weight level of the shell comprised in the water-swellable material is known, a theoretical equivalent average shell caliper may be determined as defined below.

This method calculates the average shell caliper of a shell on the particle cores of the water-swellable material herein, under the assumption that the water-swellable material is to be monodisperse and spherical (which may not be the case in practice).

Key Parameters

| INPUT Parameter | Symbol |
|---|---|
| Mass Median Particle Size of the water-swellable polymer (AGM) without shell (e.g., prior to applying the shell; also called "average diameter") | D_AGM_dry |
| Intrinsic density of the base water-swellable bulk polymer (without shell) | Rho_AGM_intrinsic |
| Intrinsic density of the material (e.g., elastomeric polymer) of the shell only | Rho_polymer shell |
| Shell Weight Fraction of the water-swellable material ( | c_shell_per_total |
| OUTPUT Parameters | |
| Average shell caliper if the water-swellable material is monodisperse and spherical | d_shell |
| Mass Median Particle Size of the particles of the water-swellable material with shells on its particles ("average diameter when shell present") | D_AGM_coated |
| Weight Ratio as of weight of shell per weight of water-swellable material without shell | c_shell_to_bulk |
| Formulas (note: in this notation: all c which are in percent have ranges of 0 to 1 which is equivalent to 0 to 100%.) | |

$$d\_shell := \frac{D\_AGM\_dry}{2} \cdot \left[ \left[ 1 + \frac{c\_shell\_per\_total}{(1 - c\_shell\_per\_total)} \cdot \frac{Rho\_AGM\_intrinsic}{Rho\_polymer\_shell} \right]^{\frac{1}{3}} - 1 \right]$$

$$D\_coated\_AGM := D\_AGM\_dry + 2 \cdot d\_shell$$

$$c\_shell\_to\_bulk := \frac{c\_shell\_per\_total}{1 - c\_shell\_per\_total}$$

Example

D_AGM_dry:=0.4mm (400$\mu$m); Rho_AGM_intrinsic:=Rho_polymer_shell:=1.5 g/cc

| | | | | | | | | |
|---|---|---|---|---|---|---|---|---|
| C_shell_per_total [%] | 1 | 2 | 5 | 10 | 20 | 30 | 40 | 50 |
| C_shell_to_bulk [%] | 1.0 | 2.0 | 5.3 | 11 | 25 | 43 | 67 | 100 |
| d_shell [$\mu$m] | 0.7 | 1.4 | 3.4 | 7.1 | 15 | 25 | 37 | 52 |
| D_Coated_AGM [$\mu$m] | 401 | 403 | 407 | 414 | 431 | 450 | 474 | 504 |

Surface tension of aqueous extract

0.50 g of the water-swellable material or polymeric particles is weighed into a small glass beaker and admixed with 40 ml of 0.9% by weight salt solution. The contents of the beaker are magnetically stirred at 500 rpm for 3 minutes and then allowed to settle for 2 minutes. Finally, the surface tension of the supernatant aqueous phase is measured with a K10-ST digital tensiometer or a comparable apparatus having a platinum plate (from Kruess). The measurement is carried out at a temperature of 23°C.

Moisture content of base polymer

The water content of the water-swellable material or polymers is determined by the EDANA (European Disposables and Nonwovens Association) recommended test method No. 430.2-02 "Moisture content".

Method to determine the water-swelling capacity of the elastomeric polymer

The weight of the polymer specimen after soaking for 3 days in an excess of deionized water at room temperature (25 °C) is taken as $W_1$. The weight of this polymer specimen before drying is taken as W0. The water swelling capacity is then calculated as follows:

$$WSC[g \ Zg] = (W_1 - W_0)/ W_0$$

The water swelling capacity is the water uptake of the polymer specimen in g water per 1 g of dry polymer. For this test method it is necessary to prepare polymer specimen that are typically not thicker than 1.0 mm for moderately swelling polymers. It may be necessary to prepare polymer films of less than 0.5 mm thickness for low swelling polymers in order to obtain equilibrium swelling after 3 days. A person skilled in the art will adjust the thickness and dry sample weight in a way to obtain equilibrium swelling conditions after 3 days.

**Claims**

1. An absorbent structure for use in an absorbent article, said absorbent structure comprising a water-swellable material said water-swellable material having an absorbent capacity of at least 20g/g (as measured in the 4-hour CCRC test) and a QUICS value of at least 15, wherein said water-swellable material comprises water-swellable particles with a core, containing water-swellable polymer(s) and a shell, containing elastomeric polymer(s) and wherein said elastomeric polymer (s) are polyetherpolyurethanes comprising alkylene oxide units on at least one of a group selected from polymer main chains and polymer side chains.

2. An absorbent structure according to claim 1, wherein said water-swellable material has a QUICS of at least 20.

3. An absorbent structure according to claim 1 wherein said water-swellable material has a CS-SFC of at least 10.

4. An absorbent structure according to claim 1 wherein said main chains comprise butylenes oxide units.

5. An absorbent structure as in claim 1, wherein said water-swellable polymers are post-crosslinked water-swellable polymers and said shells have an average shell tension of from 15 N/m to 60 N/m.

6. An absorbent structure as in claim 1, wherein said water-swellable polymers are not post-cross-linked and said shells have an average shell tension of more than 60 N/m, to 110 N/m.

7. An absorbent structure according to claim 1 wherein the water-swellable material comprises a deagglomeration aid.

8. An absorbent structure according to claim 1 comprising:

    a) a substrate layer, said substrate layer having a first surface and a second surface;
    b) a discontinuous layer of said water-swellable material, said discontinuous layer of water-swellable material comprising a first surface and a second surface,

c) a layer of thermoplastic material, comprising a first surface and a second surface,

wherein the second surface of said discontinuous layer of water-swellable material is in at least partial contact with said first surface of said substrate layer and wherein portions of said second surface of said layer of thermoplastic material are in direct contact with said first surface of said substrate layer and portions of said second surface of the said layer of thermoplastic material are in direct contact with said first surface of said discontinuous layer of water-swellable material.

9. An absorbent article that comprises the absorbent structure of claim 1.

10. An absorbent article as in claim 9, wherein the absorbent structure comprises a storage layer of the article, said absorbent structure having a density of at least 0.4 g/cm$^3$.

11. An absorbent article as in claim 10, wherein said storage layer comprises less than 20% by weight (of the water-swellable material) of absorbent fibrous material.

**Patentansprüche**

1. Absorptionsstruktur zum Gebrauch in einem Absorptionsartikel, wobei die Absorptionsstruktur ein wasserquellfähiges Material umfasst, wobei das wasserquellfähige Material ein Absorptionsvermögen von mindestens 20 g/g (wie im 4-stündigen CCRC-Test gemessen) und einen QUICS-Wert von mindestens ungefähr 15 aufweist, wobei das wasserquellfähige Material wasserquellfähige Teilchen mit einem Kern, der wasserquellfähige(s) Polymer(e) enthält, und einer Hülle, die elastomere(s) Polymer(e) enthält, aufweist und wobei das elastomere Polymer bzw. die elastomeren Polymere Polyetherpolyurethane sind, die Alkylenoxideinheiten an mindestens einer Gruppe, ausgewählt aus Polymerhauptketten und Polymerseitenketten, umfassen.

2. Absorptionsstruktur nach Anspruch 1, wobei das wasserquellfähige Material einen QUICS-Wert von mindestens 20 aufweist.

3. Absorptionsstruktur nach Anspruch 1, wobei das wasserquellfähige Material einen CS-SFC-Wert von mindestens 10 aufweist.

4. Absorptionsstruktur nach Anspruch 1, wobei die Hauptketten Butylenoxideinheiten umfassen.

5. Absorptionsstruktur nach Anspruch 1, wobei die wasserquellfähigen Polymere nachvernetzte wasserquellfähige Polymere sind und die Hüllen eine durchschnittliche Hüllenspannung von 15 N/m bis 60 N/m aufweisen.

6. Absorptionsstruktur nach Anspruch 1, wobei die wasserquellfähigen Polymere nicht nachvernetzt sind und die Hüllen eine durchschnittliche Hüllenspannung von mehr als 60 N/m bis 110 N/m aufweisen.

7. Absorptionsstruktur nach Anspruch 1, wobei das wasserquellfähige Material ein Deagglomerationshilfsmittel umfasst.

8. Absorptionsstruktur nach Anspruch 1, umfassend:

a) eine Substratschicht, wobei die Substratschicht eine erste Oberfläche und eine zweite Oberfläche aufweist,
b) eine diskontinuierliche Schicht des wasserquellfähigen Materials, wobei die diskontinuierliche Schicht aus wasserquellfähigem Material eine erste Oberfläche und eine zweite Oberfläche umfasst,
c) eine Schicht aus thermoplastischem Material, umfassend eine erste Oberfläche und eine zweite Oberfläche, wobei die zweite Oberfläche der diskontinuierlichen Schicht aus wasserquellfähigem Material mindestens teilweise in Kontakt mit der ersten Oberfläche der Substratschicht ist und wobei Abschnitte der zweiten Oberfläche der Schicht aus thermoplastischem Material in direktem Kontakt mit der ersten Oberfläche der Substratschicht sind und Abschnitte der zweiten Oberfläche der Schicht aus thermoplastischem Material in direktem Kontakt mit der ersten Oberfläche der diskontinuierlichen Schicht aus wasserquellfähigem Material sind.

9. Absorptionsartikel, der die Absorptionsstruktur nach Anspruch 1 umfasst.

**10.** Absorptionsartikel nach Anspruch 9, wobei die Absorptionsstruktur mindestens eine Speicherschicht des Artikels umfasst, wobei die Absorptionsstruktur eine Dichte von mindestens ungefähr 0,4 g/cm$^3$ aufweist.

**11.** Absorptionsartikel nach Anspruch 10, wobei die Speicherschicht zu weniger als 20 Gew.-% (des wasserquellfähigen Materials) absorbierendes Fasermaterial umfasst.

**Revendications**

**1.** Structure absorbante utilisable dans un article absorbant, ladite structure absorbante comprenant un matériau pouvant gonfler dans l'eau, ledit matériau pouvant gonfler dans l'eau possédant une capacité d'absorption d'au moins 20 g/g (telle que mesurée dans le test CRCC (capacité de rétention en centrifugeuse cylindrique) de 4 heures) et une valeur QUICS (indice de qualité de noyau-enveloppe) d'au moins environ 15, dans laquelle ledit matériau pouvant gonfler dans l'eau comprend des particules pouvant gonfler dans l'eau avec un noyau, contenant un ou des polymère(s) pouvant gonfler dans l'eau et une enveloppe, contenant un ou des polymère(s) élastomère(s) et dans laquelle ledit(lesdits) polymère(s) élastomère(s) est(sont) des polyéther-polyuréthanes comprenant des motifs oxyde d'alkylène sur au moins un élément d'un groupe choisi parmi les chaînes principales du polymère et les chaînes latérales du polymère.

**2.** Structure absorbante selon la revendication 1, dans laquelle ledit matériau pouvant gonfler dans l'eau a un indice QUICS d'au moins 20.

**3.** Structure absorbante selon la revendication 1, dans laquelle ledit matériau pouvant gonfler dans l'eau a une CS-SFC (conductivité en flux salin de noyau-enveloppe) d'au moins 10.

**4.** Structure absorbante selon la revendication 1, dans laquelle lesdites chaînes principales comprennent des motifs oxyde de butylène.

**5.** Structure absorbante selon la revendication 1, dans laquelle lesdits polymères pouvant gonfler dans l'eau sont des polymères pouvant gonfler dans l'eau post-réticulés et lesdites enveloppes ont une tension moyenne d'enveloppe allant de 15 N/m à 60 N/m.

**6.** Structure absorbante selon la revendication 1, dans laquelle lesdits polymères pouvant gonfler dans l'eau ne sont pas post-réticulés et lesdites enveloppes ont une tension moyenne d'enveloppe de plus de 60 N/m, jusqu'à 110 N/m.

**7.** Structure absorbante selon la revendication 1, dans laquelle le matériau pouvant gonfler dans l'eau comprend un auxiliaire de désagglomération.

**8.** Structure absorbante selon la revendication 1, comprenant :

a) une couche de substrat, ladite couche de substrat ayant une première surface et une deuxième surface ;
b) une couche discontinue dudit matériau pouvant gonfler dans l'eau, ladite couche discontinue de matériau pouvant gonfler dans l'eau comprenant une première surface et une deuxième surface,
c) une couche de matériau thermoplastique, comprenant une première surface et une deuxième surface, dans laquelle la deuxième surface de ladite couche discontinue de matériau pouvant gonfler dans l'eau est en contact au moins partiel avec ladite première surface de ladite couche de substrat et dans laquelle des parties de ladite deuxième surface de ladite couche de matériau thermoplastique sont en contact direct avec ladite première surface de ladite couche de substrat et des parties de ladite deuxième surface de ladite couche de matériau thermoplastique sont en contact direct avec ladite première surface de ladite couche discontinue de matériau pouvant gonfler dans l'eau.

**9.** Article absorbant qui comprend la structure absorbante selon la revendication 1.

**10.** Article absorbant selon la revendication 9, dans lequel la structure absorbante comprend une couche de stockage de l'article, ladite structure absorbante possédant une masse volumique d'au moins environ 0,4 g/cm$^3$.

**11.** Article absorbant selon la revendication 10, dans lequel ladite couche de stockage comprend moins de 20 % en poids (du matériau pouvant gonfler dans l'eau) de matériau fibreux absorbant.

# FIG.1

# FIG.2

# FIG.3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 200403625 B
- US 200403624 B
- US 200403623 B
- US 20040162536 A
- EP 1403419 A
- WO 20020192366 A
- EP 1470281 A
- EP 1470282 A
- DE 1495745 A
- WO 03050156 A
- US 3905929 A
- US 5700867 A
- US 20030195293 A

- US 4190566 A
- US 4092286 A
- US 20040214937 A
- DE 19941423 A
- EP 686650 A
- WO 0145758 A
- WO 0314300 A
- DE 12239074 A
- DE 102004051242
- US 5211985 A
- EP 0755964 A
- US 2004025836 W
- EP 640330 A

**Non-patent literature cited in the description**

- Electronic Release. Ullmann's Encyclopedia of Industrial Chemistry. 2000
- Ullmanns Encyclopadie der technischen Chemie. vol. 19, 311-313
- *Proceedings International Waterborne High Solids Coatings,* 2004, vol. 32, 299
- *International Waterborne, High-Solids, and Powder Coatings Symposium,* February 2004

- **F.L. BUCHHOLZ ; A.T. GRAHAM.** Modern Superabsorbent Polymer Technology. Wiley, 1998
- Pharmazeutische Technologie. Georg Thieme Verlag, 1989, 412-413
- Arzneiformenlehre, Wissenschaftliche. Verlagsbuchandlung mbH, 1985, 130-132
- *Drying Technology,* 2002, vol. 20 (2), 419-447
- **KUNII ; LEVENSPIEL.** *Fluidization engineering,* 1991